# EUROPEAN PATENT APPLICATION

(11) **EP 4 501 962 A1**
(43) Date of publication of application: **05.02.2025**
(21) Application number: 22934890.9
(22) Date of filing: 06.12.2022
(51) Int. Cl.: C07K 16/28, C07K 19/00, C12N 15/13, C12N 15/62, C12N 15/867, C12N 5/10, G01N 33/574, A61K 39/00, A61P 35/00, A61P 35/02

(54) **ANTI-CD7 NANOBODY, AND DERIVATIVE THEREOF AND USE THEREOF IN TUMOR THERAPY**

(30) Priority: 30.03.2022 CN 202210331478
(71) Applicant: Hebei Senlang Biotechnology Co. Ltd., Shijiazhuang, Hebei 050000 (CN)
(72) Inventor: LI, Jianqiang, Shijiazhuang, Hebei 050000 (CN); WANG, Lin, Shijiazhuang, Hebei 050000 (CN)
(74) Representative: Simmons & Simmons LLP (Munich)
(86) International application number: PCT/CN2022/136785
(87) International publication number: WO 2023/185072

(57) **Abstract**

Provided are an anti-CD7 nanobody and a derivative thereof. The derivative comprises a humanized anti-CD7 nanobody, a chimeric antigen receptor based on a single nanobody, a chimeric antigen receptor based on a double nanobody, a recombinant expression vector, an engineered host cell, a conjugate, a pharmaceutical composition, a kit, and a reagent for detecting CD7 on the cell surface. The nanobody has a good affinity to CD7, and the prepared CAR-T cells target and recognize tumor antigens and have high killing activities against tumor cells.

## Description

### CROSS-REFERENCE TO RELATED APPLICATION

The present application is entitled to priority to the following application document: invention patent application titled "ANTI-CD7 NANOBODY, AND DERIVATIVE THEREOF AND USE THEREOF IN TUMOR THERAPY" with the application number 2022103314787 submitted on March 30, 2022, the content of which is incorporated by reference in its entirety.

### TECHNICAL FIELD

The present application belongs to the technical field of biomedicines. Specifically, the present application relates to an anti-CD7 nanobody, a derivative thereof and their use in tumor therapy.

### BACKGROUND ART

CD7 antigen is a single-chain glycoprotein and a signature antigen molecule during T cell development. In addition to healthy human thymocytes, T cells and natural killer cells, hematopoietic stem and progenitor cells such as lymphoid and myeloid precursor cells also express CD7. A large number of studies have shown that CD7 molecules are expressed in most human T lymphocyte leukemia and lymphomas (Karube K, Ohshima K, Tsuchiya T, et al. Non-B, non-T neoplasms with lymphoblast morphology: further clarification and classification[J]. The American journal of surgical pathology, 2003, 27(10): 1366-1374.; Shiyong Li, Jonathan Juco, Karen P. Mann, et al. Flow Cytometry in the Differential Diagnosis of Lymphocyte-Rich Thymoma From Precursor T-Cell Acute Lymphoblastic Leukemia/Lymphoblastic Lymphoma[J]., American Journal of Clinical Pathology, 2004, 121: 268-274.) and CD7 antigen is expressed in about 10% of acute myeloid leukemia (AML) (Foon K A, Todd R F. Immunologic classification of leukemia and lymphoma[J]. Blood. 1986, 68: 1-31). In addition, when the CD7 molecule binds to its corresponding antibody, endocytosis would occur rapidly. This characteristic makes CD7 an antigen receptor suitable for targeted delivery of various functional molecules into CD7-positive cells. Relevant studies also show that there is a group of CD7-negative T lymphocytes in the human body, which group of cells can maintain the normal immune function of the human body and avoid the loss of immune function caused by using CD7 nanobody-related immune cells to eliminate all CD7-positive cells. It can be seen that targeting CD7 is a promising anti-tumor direction.

Chimeric antigen receptor modification T cells (CAR-T) and chimeric antigen receptor modification NK cells (CAR-NK) immunotherapy are the two most rapidly developed immunotherapies against cancer cells, and the effective activation of CAR-T/CAR-NK cells depends extremely on the specificity of the antibody that recognizes the tumor-associated antigen and the affinity of the antibody-antigen binding. Therefore, under the current situation that the design in the intracellular signal transduction region of CAR-T/CAR-NK cells has become mature, the design in the antigen-binding region has become the focus and key to the development of new CAR-T technology. There is a heavy chain antibody (HCAb) that naturally lacks light chains in camelids (camels, alpacas) or sharks, which HCAb only contains one heavy chain variable region and two conventional CH2 and CH3 regions. The heavy chain variable region of HCAb has comparative stability and antigen-binding activity with a heavy-chain antibody, has a size of only 2.4 × 4 nm, is the smallest fragment capable of binding an antigen and is called a single-domain antibody (Variable domain of heavy chain of heavy-chain antibody, VHH) or a nanobody. Compared with conventional antibodies, VHH single-domain antibodies have small molecular weight, high expression levels, good chemical stability, high affinity, high homology with human antibodies, and low immunogenicity. The small molecular weight makes it easy to carry out genetic engineering and construct dual- or multi-specific single-domain antibody combination to achieve the effects of multiple targets or multiple functions for one molecule. VHH has good tissue penetration and has the possibility of accessing relatively hidden targets that cannot be accessed by conventional antibodies during tumor treatment. Because of these advantages, use of single-domain antibodies as the antigen-binding region of CAR for CAR modification and CAR-T/CAR-NK cell therapy can provide a new tumor treatment strategy in this field.

### SUMMARY OF THE INVENTION

In view of the above, an objective of the present application is to provide an anti-CD7 nanobody, a derivative thereof and their use in tumor therapy.

The objective of the present application is achieved through the following technical solutions:
A first aspect of the present application provides an anti-CD7 nanobody.
Further, the nanobody is any one of VHH01, VHH03, VHH04, VHH06, VHH07, VHH08, VHH09, VHH10, VHH12, VHH13, VHH14, VHH15, VHH16, VHH17, VHH18, VHH19 or VHH20;
preferably, the amino acid sequences of CDR1, CDR2 and CDR3 of VHH01 are as shown in SEQ ID NO: 3, SEQ ID NO: 5 and SEQ ID NO: 7 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 3, SEQ ID NO: 5 and SEQ ID NO: 7 respectively;
preferably, the amino acid sequences of CDR1, CDR2 and CDR3 of VHH03 are as shown in SEQ ID NO: 11, SEQ ID NO: 13 and SEQ ID NO: 15 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 11, SEQ ID NO: 13 and SEQ ID NO: 15 respectively;
preferably, the amino acid sequences of CDR1, CDR2 and CDR3 of VHH04 are as shown in SEQ ID NO: 19, SEQ ID NO: 21 and SEQ ID NO: 23 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 19, SEQ ID NO: 21 and SEQ ID NO: 23 respectively;
preferably, the amino acid sequences of CDR1, CDR2 and CDR3 of VHH06 are as shown in SEQ ID NO: 27, SEQ ID NO: 29 and SEQ ID NO: 31 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 27, SEQ ID NO: 29 and SEQ ID NO: 31 respectively;
preferably, the amino acid sequences of CDR1, CDR2 and CDR3 of VHH07 are as shown in SEQ ID NO: 35, SEQ ID NO: 37 and SEQ ID NO: 39 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 35, SEQ ID NO: 37 and SEQ ID NO: 39 respectively;
preferably, the amino acid sequences of CDR1, CDR2 and CDR3 of VHH08 are as shown in SEQ ID NO: 43, SEQ ID NO: 45 and SEQ ID NO: 47 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 43, SEQ ID NO: 45 and SEQ ID NO: 47 respectively;
preferably, the amino acid sequences of CDR1, CDR2 and CDR3 of VHH09 are as shown in SEQ ID NO: 51, SEQ ID NO: 53 and SEQ ID NO: 55 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 51, SEQ ID NO: 53 and SEQ ID NO: 55 respectively;
preferably, the amino acid sequences of CDR1, CDR2 and CDR3 of VHH10 are as shown in SEQ ID NO: 59, SEQ ID NO: 61 and SEQ ID NO: 63 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 59, SEQ ID NO: 61 and SEQ ID NO: 63 respectively;
preferably, the amino acid sequences of CDR1, CDR2 and CDR3 of VHH12 are as shown in SEQ ID NO: 67, SEQ ID NO: 69 and SEQ ID NO: 71 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 67, SEQ ID NO: 69 and SEQ ID NO: 71 respectively;
preferably, the amino acid sequences of CDR1, CDR2 and CDR3 of VHH13 are as shown in SEQ ID NO: 75, SEQ ID NO: 77 and SEQ ID NO: 79 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 75, SEQ ID NO: 77 and SEQ ID NO: 79 respectively;
preferably, the amino acid sequences of CDR1, CDR2 and CDR3 of VHH14 are as shown in SEQ ID NO: 83, SEQ ID NO: 85 and SEQ ID NO: 87 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 83, SEQ ID NO: 85 and SEQ ID NO: 87 respectively;
preferably, the amino acid sequences of CDR1, CDR2 and CDR3 of VHH15 are as shown in SEQ ID NO: 91, SEQ ID NO: 93 and SEQ ID NO: 95 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 91, SEQ ID NO: 93 and SEQ ID NO: 95 respectively;
preferably, the amino acid sequences of CDR1, CDR2 and CDR3 of VHH16 are as shown in SEQ ID NO: 99, SEQ ID NO: 101 and SEQ ID NO: 103 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 99, SEQ ID NO: 101 and SEQ ID NO: 103 respectively;
preferably, the amino acid sequences of CDR1, CDR2 and CDR3 of VHH17 are as shown in SEQ ID NO: 107, SEQ ID NO: 109 and SEQ ID NO: 111 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 107, SEQ ID NO: 109 and SEQ ID NO: 111 respectively;
preferably, the amino acid sequences of CDR1, CDR2 and CDR3 of VHH18 are as shown in SEQ ID NO: 115, SEQ ID NO: 117 and SEQ ID NO: 119 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 115, SEQ ID NO: 117 and SEQ ID NO: 119 respectively;
preferably, the amino acid sequences of CDR1, CDR2 and CDR3 of VHH19 are as shown in SEQ ID NO: 123, SEQ ID NO: 125 and SEQ ID NO: 127 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 123, SEQ ID NO: 125 and SEQ ID NO: 127 respectively;
preferably, the amino acid sequences of CDR1, CDR2 and CDR3 of VHH20 are as shown in SEQ ID NO: 131, SEQ ID NO: 133 and SEQ ID NO: 135 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 131, SEQ ID NO: 133 and SEQ ID NO: 135 respectively;
more preferably, the nucleotide sequences of CDR1, CDR2 and CDR3 of VHH01 are as shown in SEQ ID NO: 4, SEQ ID NO: 6 and SEQ ID NO: 8 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 4, SEQ ID NO: 6 and SEQ ID NO: 8 respectively;
more preferably, the nucleotide sequences of CDR1, CDR2 and CDR3 of VHH03 are as shown in SEQ ID NO: 12, SEQ ID NO: 14 and SEQ ID NO: 16 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 12, SEQ ID NO: 14 and SEQ ID NO: 16 respectively;
more preferably, the nucleotide sequences of CDR1, CDR2 and CDR3 of VHH04 are as shown in SEQ ID NO: 20, SEQ ID NO: 22 and SEQ ID NO: 24 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 20, SEQ ID NO: 22 and SEQ ID NO: 24 respectively;
more preferably, the nucleotide sequences of CDR1, CDR2 and CDR3 of VHH06 are as shown in SEQ ID NO: 28, SEQ ID NO: 30 and SEQ ID NO: 32 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 28, SEQ ID NO: 30 and SEQ ID NO: 32 respectively;
more preferably, the nucleotide sequences of CDR1, CDR2 and CDR3 of VHH07 are as shown in SEQ ID NO: 36, SEQ ID NO: 38 and SEQ ID NO: 40 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 36, SEQ ID NO: 38 and SEQ ID NO: 40 respectively;
more preferably, the nucleotide sequences of CDR1, CDR2 and CDR3 of VHH08 are as shown in SEQ ID NO: 44, SEQ ID NO: 46 and SEQ ID NO: 48 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 44, SEQ ID NO: 46 and SEQ ID NO: 48 respectively;
more preferably, the nucleotide sequences of CDR1, CDR2 and CDR3 of VHH09 are as shown in SEQ ID NO: 52, SEQ ID NO: 54 and SEQ ID NO: 56 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 52, SEQ ID NO: 54 and SEQ ID NO: 56 respectively;
more preferably, the nucleotide sequences of CDR1, CDR2 and CDR3 of VHH10 are as shown in SEQ ID NO: 60, SEQ ID NO: 62 and SEQ ID NO: 64 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 60, SEQ ID NO: 62 and SEQ ID NO: 64 respectively;
more preferably, the nucleotide sequences of CDR1, CDR2 and CDR3 of VHH12 are as shown in SEQ ID NO: 68, SEQ ID NO: 70 and SEQ ID NO: 72 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 68, SEQ ID NO: 70 and SEQ ID NO: 72 respectively;
more preferably, the nucleotide sequences of CDR1, CDR2 and CDR3 of VHH13 are as shown in SEQ ID NO: 76, SEQ ID NO: 78 and SEQ ID NO: 80 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 76, SEQ ID NO: 78 and SEQ ID NO: 80 respectively;
more preferably, the nucleotide sequences of CDR1, CDR2 and CDR3 of VHH14 are as shown in SEQ ID NO: 84, SEQ ID NO: 86 and SEQ ID NO: 88 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 84, SEQ ID NO: 86 and SEQ ID NO: 88 respectively;
more preferably, the nucleotide sequences of CDR1, CDR2 and CDR3 of VHH15 are as shown in SEQ ID NO: 92, SEQ ID NO: 94 and SEQ ID NO: 96 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 92, SEQ ID NO: 94 and SEQ ID NO: 96 respectively;
more preferably, the nucleotide sequences of CDR1, CDR2 and CDR3 of VHH16 are as shown in SEQ ID NO: 100, SEQ ID NO: 102 and SEQ ID NO: 104 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 100, SEQ ID NO: 102 and SEQ ID NO: 104 respectively;
more preferably, the nucleotide sequences of CDR1, CDR2 and CDR3 of VHH17 are as shown in SEQ ID NO: 108, SEQ ID NO: 110 and SEQ ID NO: 112 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 108, SEQ ID NO: 110 and SEQ ID NO: 112 respectively;
more preferably, the nucleotide sequences of CDR1, CDR2 and CDR3 of VHH18 are as shown in SEQ ID NO: 116, SEQ ID NO: 118 and SEQ ID NO: 120 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 116, SEQ ID NO: 118 and SEQ ID NO: 120 respectively;
more preferably, the nucleotide sequences of CDR1, CDR2 and CDR3 of VHH19 are as shown in SEQ ID NO: 124, SEQ ID NO: 126 and SEQ ID NO: 128 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 124, SEQ ID NO: 126 and SEQ ID NO: 128 respectively;
more preferably, the nucleotide sequences of CDR1, CDR2 and CDR3 of VHH20 are as shown in SEQ ID NO: 132, SEQ ID NO: 134 and SEQ ID NO: 136 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 132, SEQ ID NO: 134 and SEQ ID NO: 136 respectively;
most preferably, the amino acid sequences of VHH01, VHH03, VHH04, VHH06, VHH07, VHH08, VHH09, VHH10, VHH12, VHH13, VHH14, VHH15, VHH16, VHH17, VHH18, VHH19, and VHH20 are as shown in SEQ ID NO: 1, SEQ ID NO: 9, SEQ ID NO: 17, SEQ ID NO: 25, SEQ ID NO: 33, SEQ ID NO: 41, SEQ ID NO: 49, SEQ ID NO: 57, SEQ ID NO: 65, SEQ ID NO: 73, SEQ ID NO: 81, SEQ ID NO: 89, SEQ ID NO: 97, SEQ ID NO: 105, SEQ ID NO: 113, SEQ ID NO: 121, and SEQ ID NO: 129 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 1, SEQ ID NO: 9, SEQ ID NO: 17, SEQ ID NO: 25, SEQ ID NO: 33, SEQ ID NO: 41, SEQ ID NO: 49, SEQ ID NO: 57, SEQ ID NO: 65, SEQ ID NO: 73, SEQ ID NO: 81, SEQ ID NO: 89, SEQ ID NO: 97, SEQ ID NO: 105, SEQ ID NO: 113, SEQ ID NO: 121, and SEQ ID NO : 129 respectively;
most preferably, the nucleotide sequences of VHH01, VHH03, VHH04, VHH06, VHH07, VHH08, VHH09, VHH10, VHH12, VHH13, VHH14, VHH15, VHH16, VHH17, VHH18, VHH19, and VHH20 are as shown in SEQ ID NO: 2, SEQ ID NO: 10, SEQ ID NO: 18, SEQ ID NO: 26, SEQ ID NO: 34, SEQ ID NO: 42, SEQ ID NO: 50, SEQ ID NO: 58, SEQ ID NO: 66, SEQ ID NO: 74, SEQ ID NO: 82, SEQ ID NO: 90, SEQ ID NO: 98, SEQ ID NO: 106, SEQ ID NO: 114, SEQ ID NO: 122, and SEQ ID NO: 130 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 2, SEQ ID NO: 10, SEQ ID NO: 18, SEQ ID NO: 26, SEQ ID NO: 34, SEQ ID NO: 42, SEQ ID NO: 50, SEQ ID NO: 58, SEQ ID NO: 66, SEQ ID NO: 74, SEQ ID NO: 82, SEQ ID NO: 90, SEQ ID NO: 98, SEQ ID NO: 106, SEQ ID NO: 114, SEQ ID NO: 122, and SEQ ID NO : 130 respectively.

Further, nano-antibodies obtained from any combination of amino acid sequences or nucleotide sequences of CDR1, CDR2 and CDR3 of one or more of VHH01, VHH03, VHH04, VHH06, VHH07, VHH08, VHH09, VHH10, VHH12, VHH13, VHH14, VHH15, VHH16, VHH17, VHH18, VHH19 or VHH20 are also within the scope of the present application.

Further, CD7 is a very stable marker on the surface of T cells. Both naive T cells and mature T cells express CD7, and thus both patients with naive T-cell tumor (T-ALL/LBL/NKT cell leukemia) and patients with mature T-cell tumor (peripheral T-cell lymphoma, NKT cell lymphoma, and anaplastic large cell lymphoma) basically express CD7 at high level. Therefore, CAR-T targeting this target to treat T-lineage blood tumors is one of the most rapidly developing technologies in clinical practice at present.

A second aspect of the present application provides a humanized anti-CD7 nanobody.

Further, the humanized anti-CD7 nanobody is obtained by humanizing a residue at a key position in the nanobody using the universal humanization framework h-NbBcII10FGLA as a reference via alignment with DP-47;
preferably, the humanized anti-CD7 nanobody is any one of hVHH01, hVHH03, hVHH04, hVHH06, hVHH07, hVHH08, hVHH09, hVHH10, hVHH12, hVHH13, hVHH14, hVHH15, hVHH16, hVHH17, hVHH18, hVHH19 or hVHH20;
more preferably, the amino acid sequences of CDR1, CDR2 and CDR3 of hVHH06 are as shown in SEQ ID NO: 139, SEQ ID NO: 141 and SEQ ID NO: 143 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 139, SEQ ID NO: 141 and SEQ ID NO: 143 respectively;
most preferably, the nucleotide sequences of CDR1, CDR2 and CDR3 of hVHH06 are as shown in SEQ ID NO: 140, SEQ ID NO: 142 and SEQ ID NO: 144 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 140, SEQ ID NO: 142 and SEQ ID NO: 144 respectively;
most preferably, the amino acid sequence of hVHH06 is as shown in SEQ ID NO: 137 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 137;
most preferably, the nucleotide sequence of hVHH06 is as shown in SEQ ID NO: 138 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 138.

A third aspect of the present application provides a chimeric antigen receptor based on a single nanobody.

Further, the chimeric antigen receptor includes any one selected from the nanobody according to the first aspect of the present application or the humanized anti-CD7 nanobody according to the second aspect of the present application;
preferably, the chimeric antigen receptor further comprises a transmembrane domain;
preferably, the chimeric antigen receptor further comprises an intracellular signaling domain;
preferably, the chimeric antigen receptor further comprises a hinge region;
preferably, the chimeric antigen receptor further comprises a signal peptide;
preferably, the chimeric antigen receptor further comprises a costimulatory signaling domain;
preferably, the chimeric antigen receptor further comprises a promoter EF1α;
preferably, the chimeric antigen receptor further comprises a self-cleaving peptide T2A;
preferably, the chimeric antigen receptor further comprises a detection tag/auxiliary functional element tEGFR;
more preferably, the transmembrane domain comprises the transmembrane domain of the following molecules: CD8α, CD28, IgG1, IgG4, 4-1BB, PD-1, CD34, OX40, CD3ε, IL-2 receptor, IL-7 receptor, IL-11 receptor;
more preferably, the intracellular signaling domain comprises the intracellular signaling domain of the following molecules: CD3ζ, FcRγ, FcRβ, CD3γ, CD3δ, CD3ε, TCRζ, CD4, CD5, CD8, CD21, CD22, CD79a, CD79b, CD278, FcεRI, DAP10, DAP12, CD66d;
more preferably, the hinge region comprises the hinge region of the following molecules: CD8α, CD28, IgG1, IgG4, 4-1BB, PD-1, CD34, OX40, CD3ε, IL-2 receptor, IL-7 receptor, IL-11 receptor;
more preferably, the signal peptide comprises the signal peptide of the following molecules: alpha and beta chains of T cell receptor, CD3ζ, CD3ε, CD4, CD5, CD8, CD9, CD28, CD16, CD22, CD33, CD37, CD45, CD64, CD80, CD86, CD134, CD137, CD154, GITR, GM -CSF;
more preferably, the costimulatory signaling domain comprises the costimulatory signaling domain of the following molecules: 4-1BB(CD137), CD27, CD19, CD4, CD28, ICOS(CD278), CD8α, CD8β, BAFFR, HVEM, LIGHT, KIRDS2, SLAMF7, NKp30, NKp46, CD40, CDS, ICAM-1, B7-H3, OX40, DR3, GITR, CD30, TIM1, CD2, CD7, CD226;
more preferably, the promoter is not limited to EF1α promoter, but further comprises: a CMV promoter, a EFS promoter, a CAG promoter, a CBh promoter, a SFFV promoter, a MSCV promoter, a SV40 promoter, a mPGK promoter, a hPGK promoter, and a UBC promoter;
more preferably, the self-cleaving peptide is not limited to T2A, but further comprises: P2A, E2A, and F2A;
more preferably, the detection tag/auxiliary functional element is not limited to tEGFR, but further comprises: tCD34, tCD19, tCD20, tCD22, an immune checkpoint inhibitor (CTLA-4, PD-1/PD-L1, LAG-3, TIM-3, TIGIT, CD226, CD155, CD47, B7-H3, B7-H4) nanobody, a cytokine and its receptor (IL2, IL2 receptor, IL7, IL7 receptor, IL15, and IL15 receptor);
most preferably, the transmembrane domain is a CD8α transmembrane domain;
most preferably, the intracellular signaling domain is a CD3ζ intracellular signaling domain;
most preferably, the hinge region is a CD8α hinge region;
most preferably, the costimulatory signaling domain is a 4-1BB costimulatory signaling domain;
most preferably, the amino acid sequence of the signal peptide is as shown in SEQ ID NO: 145 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 145;
most preferably, the nucleotide sequence of the signal peptide is as shown in SEQ ID NO: 146 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 146;
most preferably, the amino acid sequence of the CD8α hinge region is as shown in SEQ ID NO: 147 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 147;
most preferably, the nucleotide sequence of the CD8α hinge region is as shown in SEQ ID NO: 148 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 148;
most preferably, the amino acid sequence of the CD8α transmembrane domain is as shown in SEQ ID NO: 149 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 149;
most preferably, the nucleotide sequence of the CD8α transmembrane domain is as shown in SEQ ID NO: 150 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 150;
most preferably, the amino acid sequence of the 4-1BB costimulatory signaling domain is as shown in SEQ ID NO: 151 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 151;
most preferably, the nucleotide sequence of the 4-1BB costimulatory signaling domain is as shown in SEQ ID NO: 152 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 152;
most preferably, the amino acid sequence of the CD3ζ intracellular signaling domain is as shown in SEQ ID NO: 153 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 153;
most preferably, the nucleotide sequence of the CD3ζ intracellular signaling domain is as shown in SEQ ID NO: 154 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 154;
most preferably, the amino acid sequence of the T2A is as shown in SEQ ID NO: 155 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 155;
most preferably, the nucleotide sequence of the T2A is as shown in SEQ ID NO: 156 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 156;
most preferably, the nucleotide sequence of the EF1α is as shown in SEQ ID NO: 157 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 157;
most preferably, the amino acid sequence of the tEGFR signal peptide is as shown in SEQ ID NO: 158 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 158;
most preferably, the nucleotide sequence of the tEGFR signal peptide is as shown in SEQ ID NO: 159 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 159;
most preferably, the amino acid sequence of the tEGFR is as shown in SEQ ID NO: 160 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 160;
most preferably, the nucleotide sequence of the tEGFR is as shown in SEQ ID NO: 161 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 161;
most preferably, the chimeric antigen receptor is obtained by sequentially linking EF1α, a signal peptide, any one selected from the nanobody according to the first aspect of the present application or the humanized anti-CD7 nanobody according to the second aspect of the present application, a CD8α hinge region, a CD8α transmembrane domain, a 4-1BB costimulatory signaling domain, a CD3ζ intracellular signaling domain, T2A, a tEGFR signal peptide, and tEGFR in series.

A fourth aspect of the present application provides a chimeric antigen receptor based on a double nanobody.

Further, the chimeric antigen receptor includes any two selected from the nanobody according to the first aspect of the present application or the humanized anti-CD7 nanobody according to the second aspect of the present application;
preferably, the chimeric antigen receptor further comprises a transmembrane domain;
preferably, the chimeric antigen receptor further comprises an intracellular signaling domain;
preferably, the chimeric antigen receptor further comprises a hinge region;
preferably, the chimeric antigen receptor further comprises a signal peptide;
preferably, the chimeric antigen receptor further comprises a costimulatory signaling domain;
preferably, the chimeric antigen receptor further comprises a promoter EF1α;
preferably, the chimeric antigen receptor further comprises a self-cleaving peptide T2A;
preferably, the chimeric antigen receptor further comprises a detection tag/auxiliary functional element tEGFR;
more preferably, the transmembrane domain comprises the transmembrane domain of the following molecules: CD8α, CD28, IgG1, IgG4, 4-1BB, PD-1, CD34, OX40, CD3ε, IL-2 receptor, IL-7 receptor, IL-11 receptor;
more preferably, the intracellular signaling domain comprises the intracellular signaling domain of the following molecules: CD3ζ, FcRγ, FcRβ, CD3γ, CD3δ, CD3ε, TCRζ, CD4, CD5, CD8, CD21, CD22, CD79a, CD79b, CD278, FcεRI, DAP10, DAP12, CD66d;
more preferably, the hinge region comprises the hinge region of the following molecules: CD8α, CD28, IgG1, IgG4, 4-1BB, PD-1, CD34, OX40, CD3ε, IL-2 receptor, IL-7 receptor, IL-11 receptor;
more preferably, the signal peptide comprises the signal peptide of the following molecules: alpha and beta chains of T cell receptor, CD3ζ, CD3ε, CD4, CD5, CD8, CD9, CD28, CD16, CD22, CD33, CD37, CD45, CD64, CD80, CD86, CD134, CD137, CD154, GITR, GM-CSF;
more preferably, the costimulatory signaling domain comprises the costimulatory signaling domain of the following molecules: 4-1BB(CD137), CD27, CD19, CD4, CD28, ICOS(CD278), CD8α, CD8β, BAFFR, HVEM, LIGHT, KIRDS2, SLAMF7, NKp30, NKp46, CD40, CDS, ICAM-1, B7-H3, OX40, DR3, GITR, CD30, TIM1, CD2, CD7, CD226;
more preferably, the promoter is not limited to EF1α promoter, but further comprises: a CMV promoter, a EFS promoter, a CAG promoter, a CBh promoter, a SFFV promoter, a MSCV promoter, a SV40 promoter, a mPGK promoter, a hPGK promoter, and a UBC promoter;
more preferably, the self-cleaving peptide is not limited to T2A, but further comprises: P2A, E2A, and F2A;
more preferably, the detection tag/auxiliary functional element is not limited to tEGFR, but further comprises: tCD34, tCD19, tCD20, tCD22, an immune checkpoint inhibitor (CTLA-4, PD-1/PD-L1, LAG-3, TIM-3, TIGIT, CD226, CD155, CD47, B7-H3, B7-H4) nanobody, a cytokine and its receptor (IL2, IL2 receptor, IL7, IL7 receptor, IL15, and IL15 receptor);
most preferably, the transmembrane domain is a CD8α transmembrane domain;
most preferably, the intracellular signaling domain is a CD3ζ intracellular signaling domain;
most preferably, the hinge region is a CD8α hinge region;
most preferably, the costimulatory signaling domain is a 4-1BB costimulatory signaling domain;
most preferably, any two nanobodies selected from the nanobody according to the first aspect of the present application or the humanized anti-CD7 nanobody according to the second aspect of the present application are linked through a linking peptide Linker;
most preferably, the chimeric antigen receptor is obtained by sequentially linking EF1α, a signal peptide, any two selected from the nanobody according to the first aspect of the present application or the humanized anti-CD7 nanobody according to the second aspect of the present application, a CD8α hinge region, a CD8α transmembrane domain, a 4-1BB costimulatory signaling domain, a CD3ζ intracellular signaling domain, T2A, a tEGFR signal peptide, and tEGFR in series;
most preferably, the chimeric antigen receptor is obtained by sequentially linking EF1α, a signal peptide, any one selected from the nanobody according to the first aspect of the present application, Linker, any one selected from the nanobody according to the first aspect of the present application, a CD8α hinge region, a CD8α transmembrane domain, a 4-1BB costimulatory signaling domain, a CD3ζ intracellular signaling domain, T2A, a tEGFR signal peptide, and tEGFR in series;
most preferably, any one selected from the nanobody according to the first aspect of the present application is VHH03, VHH06, VHH10, or VHH12;
most preferably, the chimeric antigen receptor is obtained by sequentially linking EF1α, a signal peptide, VHH06 as described in the first aspect of the present application, Linker, VHH03 as described in the first aspect of the present application, a CD8α hinge region, a CD8α transmembrane domain, a 4-1BB costimulatory signaling domain, a CD3ζ intracellular signaling domain, T2A, a tEGFR signal peptide, and tEGFR in series;
most preferably, the chimeric antigen receptor is obtained by sequentially linking EF1α, a signal peptide, VHH06 as described in the first aspect of the present application, Linker, VHH12 as described in the first aspect of the present application, a CD8α hinge region, a CD8α transmembrane domain, a 4-1BB costimulatory signaling domain, a CD3ζ intracellular signaling domain, T2A, a tEGFR signal peptide, and tEGFR in series;
most preferably, the chimeric antigen receptor is obtained by sequentially linking EF1α, a signal peptide, VHH10 as described in the first aspect of the present application, Linker, VHH12 as described in the first aspect of the present application, a CD8α hinge region, a CD8α transmembrane domain, a 4-1BB costimulatory signaling domain, a CD3ζ intracellular signaling domain, T2A, a tEGFR signal peptide, and tEGFR in series;
most preferably, the chimeric antigen receptor is obtained by sequentially linking EF1α, a signal peptide, VHH10 as described in the first aspect of the present application, Linker, VHH10 as described in the first aspect of the present application, a CD8α hinge region, a CD8α transmembrane domain, a 4-1BB costimulatory signaling domain, a CD3ζ intracellular signaling domain, T2A, a tEGFR signal peptide, and tEGFR in series;
most preferably, the chimeric antigen receptor is obtained by sequentially linking EF1α, a signal peptide, VHH12 as described in the first aspect of the present application, Linker, VHH12 as described in the first aspect of the present application, a CD8α hinge region, a CD8α transmembrane domain, a 4-1BB costimulatory signaling domain, a CD3ζ intracellular signaling domain, T2A, a tEGFR signal peptide, and tEGFR in series;
most preferably, the amino acid sequence of the signal peptide is as shown in SEQ ID NO: 145 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 145;
most preferably, the nucleotide sequence of the signal peptide is as shown in SEQ ID NO: 146 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 146;
most preferably, the amino acid sequence of the CD8α hinge region is as shown in SEQ ID NO: 147 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 147;
most preferably, the nucleotide sequence of the CD8α hinge region is as shown in SEQ ID NO: 148 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 148;
most preferably, the amino acid sequence of the CD8α transmembrane domain is as shown in SEQ ID NO: 149 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 149;
most preferably, the nucleotide sequence of the CD8α transmembrane domain is as shown in SEQ ID NO: 150 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 150;
most preferably, the amino acid sequence of the 4-1BB costimulatory signaling domain is as shown in SEQ ID NO: 151 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 151;
most preferably, the nucleotide sequence of the 4-1BB costimulatory signaling domain is as shown in SEQ ID NO: 152 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 152;
most preferably, the amino acid sequence of the CD3ζ intracellular signaling domain is as shown in SEQ ID NO: 153 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 153;
most preferably, the nucleotide sequence of the CD3ζ intracellular signaling domain is as shown in SEQ ID NO: 154 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 154;
most preferably, the amino acid sequence of the T2A is as shown in SEQ ID NO: 155 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 155;
most preferably, the nucleotide sequence of the T2A is as shown in SEQ ID NO: 156 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 156;
most preferably, the nucleotide sequence of the EF1α is as shown in SEQ ID NO: 157 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 157;
most preferably, the amino acid sequence of the tEGFR signal peptide is as shown in SEQ ID NO: 158 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 158;
most preferably, the nucleotide sequence of the tEGFR signal peptide is as shown in SEQ ID NO: 159 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 159;
most preferably, the amino acid sequence of the tEGFR is as shown in SEQ ID NO: 160 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 160;
most preferably, the nucleotide sequence of the tEGFR is as shown in SEQ ID NO: 161 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 161.

Also expressly included within the scope of the present application are functional portions of the chimeric antigen receptors (CARs) disclosed herein. The term "functional portion" when used in reference to a chimeric antigen receptor (CAR) refers to any part or fragment of one or more of the CARs disclosed herein, which part or fragment retains the biological activity of the CAR of which it is a part (the parent CAR). Functional portions encompass, for example, those parts of a CAR that retain the ability to recognize target cells, or detect, treat, or prevent a disease, to a similar extent, the same extent, or to a higher extent, as the parent CAR. In reference to the parent CAR, the functional portion can comprise, for instance, about 10%, 25%, 30%, 50%, 68%, 80%, 90%, 95%, or more, of the parent CAR.

The functional portion can comprise additional amino acids at the amino or carboxy terminus of the portion, or at both termini, which additional amino acids are not found in the amino acid sequence of the parent CAR. Desirably, the additional amino acids do not interfere with the biological function of the functional portion, e.g., recognize target cells, detect cancer, treat or prevent cancer, etc. More desirably, the additional amino acids enhance the biological activity, as compared to the biological activity of the parent CAR.

Included in the scope of the disclosure are functional variants of the CARs disclosed herein. The term "functional variant" as used herein refers to a CAR, polypeptide, or protein having substantial or significant sequence identity or similarity to a parent CAR, which functional variant retains the biological activity of the CAR of which it is a variant. Functional variants encompass, for example, those variants of the CAR described herein (the parent CAR) that retain the ability to recognize target cells to a similar extent, the same extent, or to a higher extent, as the parent CAR. In reference to the parent CAR, the functional variant can, for instance, be at least about 30%, 50%, 75%, 80%, 90%, 98% or more identical in amino acid sequence to the parent CAR.

Afunctional variant can, for example, comprise the amino acid sequence of the parent CAR with at least one conservative amino acid substitution. Alternatively or additionally, the functional variants can comprise the amino acid sequence of the parent CAR with at least one non-conservative amino acid substitution. In this case, it is preferable for the non-conservative amino acid substitution to not interfere with or inhibit the biological activity of the functional variant. The non-conservative amino acid substitution may enhance the biological activity of the functional variant, such that the biological activity of the functional variant is increased as compared to the parent CAR.

Amino acid substitutions of the CARs are preferably conservative amino acid substitutions. Conservative amino acid substitutions are known in the art, and include amino acid substitutions in which one amino acid having certain physical and/or chemical properties is exchanged for another amino acid that has the same or similar chemical or physical properties. For instance, the conservative amino acid substitution can be an acidic/negatively charged polar amino acid substituted for another acidic/negatively charged polar amino acid (e.g., Asp or Glu), an amino acid with a nonpolar side chain substituted for another amino acid with a nonpolar side chain (e.g., Ala, Gly, Val, He, Leu, Met, Phe, Pro, Trp, Cys, Val, etc.), a basic/positively charged polar amino acid substituted for another basic/positively charged polar amino acid (e.g. Lys, His, Arg, etc.), an uncharged amino acid with a polar side chain substituted for another uncharged amino acid with a polar side chain (e.g., Asn, Gin, Ser, Thr, Tyr, etc.), an amino acid with a beta- branched side-chain substituted for another amino acid with a beta-branched side-chain (e.g., He, Thr, and Val), an amino acid with an aromatic side-chain substituted for another amino acid with an aromatic side chain (e.g., His, Phe, Trp, and Tyr), etc.

The CAR can consist essentially of the specified amino acid sequence or sequences described herein, such that other components, e.g., other amino acids, do not materially change the biological activity of the functional variant. The CARs (including functional portions and functional variants) can be of any length, i.e., can comprise any number of amino acids, provided that the CARs (or functional portions or functional variants thereof) retain their biological activity, e.g., the ability to specifically bind to antigen, detect diseased cells in a mammal, or treat or prevent disease in a mammal, etc. For example, the CAR can be about 50 to about 5000 amino acids long, such as 50, 70, 75, 100, 125, 150, 175, 200, 300, 400, 500, 600, 700, 800, 900, 1000 or more amino acids in length.

The CARs (including functional portions and functional variants of the present application) can comprise synthetic amino acids in place of one or more naturally-occurring amino acids. Such synthetic amino acids are known in the art, and include, for example, aminocyclohexane carboxylic acid, norleucine, -amino n-decanoic acid, homoserine, S-acetylaminomethyl-cysteine, trans-3- and trans-4-hydroxyproline, 4-aminophenylalanine, 4- nitrophenylalanine, 4-chlorophenylalanine, 4-carboxyphenylalanine, β-phenylserine β-hydroxyphenylalanine, phenylglycine, α-naphthylalanine, cyclohexylalanine, cyclohexylglycine, indoline-2-carboxylic acid, l,2,3,4-tetrahydroisoquinoline-3-carboxylic acid, aminomalonic acid, aminomalonic acid monoamide, N'-benzyl-N'-methyl-lysine, N',N'-dibenzyl-lysine, 6-hydroxylysine, ornithine, - aminocyclopentane carboxylic acid, α-aminocyclohexane carboxylic acid, α-aminocycloheptane carboxylic acid, α-(2-amino-2-norbomane)-carboxylic acid, γ-diaminobutyric acid, β-diaminopropionic acid, homophenylalanine, and a-tert-butylglycine.

The CARs (including functional portions and functional variants) can be glycosylated, amidated, carboxylated, phosphorylated, esterified, N-acylated, cyclized via, e.g., a disulfide bridge, or converted into an acid addition salt and/or optionally dimerized or polymerized, or conjugated.

A fifth aspect of the present application provides a nucleic acid molecule.

Further, the nucleic acid molecule comprises a nucleotide sequence encoding the nanobody according to the first aspect of the present application, the humanized anti-CD7 nanobody according to the second aspect of the present application, the chimeric antigen receptor according to the third aspect of the present application, or the chimeric antigen receptor according to the fourth aspect of the present application;
preferably, the nucleotide sequence is as shown in SEQ ID NO: 2, SEQ ID NO: 10, SEQ ID NO: 18, SEQ ID NO: 26, SEQ ID NO: 34, SEQ ID NO: 42, SEQ ID NO: 50. SEQ ID NO: 58, SEQ ID NO: 66, SEQ ID NO: 74, SEQ ID NO: 82, SEQ ID NO: 90, SEQ ID NO: 98, SEQ ID NO: 106, SEQ ID NO: 114, SEQ ID NO: 122, or SEQ ID NO: 130 or a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 2, SEQ ID NO: 10, SEQ ID NO: 18, SEQ ID NO: 26, SEQ ID NO: 34, SEQ ID NO: 42, SEQ ID NO: 50, SEQ ID NO: 58, SEQ ID NO: 66, SEQ ID NO: 74, SEQ ID NO: 82, SEQ ID NO: 90, SEQ ID NO: 98, SEQ ID NO: 106, SEQ ID NO: 114, SEQ ID NO: 122, or SEQ ID NO: 130.

"Nucleic acid molecule" or "Nucleic acid" as used herein can contain natural, non-natural or altered nucleotides, and it can contain a natural, non-natural or altered internucleotide linkage, such as a phosphoroamidate linkage or a phosphorothioate linkage, instead of the phosphodiester found between the nucleotides of an unmodified oligonucleotide. In some embodiments, the nucleic acid does not comprise any insertions, deletions, inversions, and/or substitutions. However, it may be suitable in some instances, as discussed herein, for the nucleic acid to comprise one or more insertions, deletions, inversions, and/or substitutions, and therefore, the nucleic acid formed via these insertions, deletions, inversions and/or substitutions is also within the scope of the present application.

In a specific embodiment of the present application, the nucleic acid molecule comprises a nucleic acid molecule that can be chemically synthesized using naturally occurring nucleotides or variously modified nucleotides designed to increase the biological stability of the molecules or to increase the physical stability of the duplex formed upon hybridization (e.g., phosphorothioate derivatives and acridine substituted nucleotides). Examples of modified nucleotides that can be used to generate the nucleic acids include, but are not limited to, 5-fluorouracil, 5-bromouracil, 5-chlorouracil, 5-iodouracil, hypoxanthine, xanthine, 4-acetylcytosine, 5-(carboxyhydroxymethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1 -methylguanine, 1 - methylinosine, 2,2-dimethylguanine, 2-methyladenine, 2-methylguanine, 3-methylcytosine, 5-methylcytosine, N6-substituted adenine, 7-methylguanine, 5-methylaminomethyluracil, 5-methoxyaminomethyl-2-thiouracil, beta-D-mannosylqueosine, 5'-methoxycarboxymethyluracil, 5- methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, 3- (3-amino-3-N-2-carboxypropyl) uracil, and 2,6-diaminopurine.

A sixth aspect of the present application provides a recombinant expression vector.

Further, the recombinant expression vector comprises the nucleic acid molecule according to the fifth aspect of the present application;
preferably, the expression vector comprises a DNA vector, an RNA vector, a plasmid, a transposon vector, a CRISPR/Cas9 vector, or a viral vector;
more preferably, the viral vector comprises a lentiviral vector, an adenoviral vector, a retroviral vector.

In an embodiment, the recombinant expression vector can be any suitable recombinant expression vector, and can be used to transform or transfect any suitable host cell. Suitable vectors include those designed for propagation and expansion or for expression or both, such as plasmids and viruses. The vector can be selected from the group consisting of the pUC series (Fermentas Life Sciences, Glen Bumie, MD), the pBluescript series (Stratagene, LaJolla, CA), the pET series (Novagen, Madison, WI), the pGEX series (Pharmacia Biotech, Uppsala, Sweden), and the pEX series (Clontech, Palo Alto, CA).

Bacteriophage vectors, such as λZapII (Stratagene), EMBL4, and λNMI149, also can be used. Examples of plant expression vectors include pBIO1, pBI101.2, pBHO1.3, pBI121 and pBIN19 (Clontech). Examples of animal expression vectors include pEUK-Cl, pMAM, and pMAMneo (Clontech). The recombinant expression vector may be a viral vector, e.g., a retroviral vector or a lentiviral vector. A lentiviral vector is a vector derived from at least a portion of a lentivirus genome, including especially a self-inactivating lentiviral vector as provided in Milone et al, Mol. Ther. 17(8): 1453-1464 (2009). Other examples of lentivirus vectors that may be used in the clinic, include, for example, and not by way of limitation, the LENTIVECTOR.RTM. gene delivery technology from Oxford BioMedica plc, the LENTIMAX.TM. vector system from Lentigen and the like. Nonclinical types of lentiviral vectors are also available and would be known to one skilled in the art.

The recombinant expression vector may comprise regulatory sequences, such as transcription and translation initiation and termination codons, which are specific to the type of host cell (e.g., bacterium, fungus, plant, or animal) into which the vector is to be introduced, as appropriate, and taking into consideration whether the vector is DNA- or RNA-based. The recombinant expression vector may comprise restriction sites to facilitate cloning.

The recombinant expression vector can include one or more marker genes, which allow for selection of transformed or transfected host cells. Marker genes include biocide resistance, e.g., resistance to antibiotics, heavy metals, etc., complementation in an auxotrophic host to provide prototrophy, and the like. Suitable marker genes for the inventive expression vectors include, for instance, neomycin/G4l8 resistance genes, hygromycin resistance genes, histidinol resistance genes, tetracycline resistance genes, ampicillin resistance genes, a kanamycin resistance gene, a puromycin resistance genes, *etc.*

The recombinant expression vector can comprise a native or nonnative promoter operably linked to the nucleotide sequence encoding the CAR (including functional portions and functional variants thereof), or to the nucleotide sequence which is complementary to or which hybridizes to the nucleotide sequence encoding the CAR. The selection of promoters, e.g., strong, weak, inducible, tissue-specific and developmental-specific, is within the ordinary skill of the artisan. Similarly, the combining of a nucleotide sequence with a promoter is also within the skill of the artisan. The promoter can be a non-viral promoter or a viral promoter, e.g., a type III promoter (U6 promoter, H1 promoter), a mammalian constitutive promoter (a universal promoter): a CMV (cytomegalovirus) promoter; a EF1A (elongation factor-1α) promoter; a EFS promoter; a CAG (composed of cytomegalovirus enhancer and chicken β-actin promoter) promoter; a CBh promoter; a SFFV promoter; a MSCV promoter; a SV40 (derived from simian virus) promoter; a mPGK promoter; a hPGK (phosphoglycerate kinase) promoter; a UBC (ubiquitin C) promoter, a RSV promoter, or a promoter found in the long terminal repeat of murine stem cell virus.

The recombinant expression vectors can be designed for either transient expression, for stable expression, or for both. Also, the recombinant expression vectors can be made for constitutive expression or for inducible expression.

Further, the recombinant expression vectors can be made to include a suicide gene. As used herein, the term "suicide gene" refers to a gene that causes the cell expressing the suicide gene to die. The suicide gene can be a gene that confers sensitivity to an agent, e.g., a drug, upon the cell in which the gene is expressed, and causes the cell to die when the cell is contacted with or exposed to the agent. Suicide genes are known in the art (see, for example, Suicide Gene Therapy: Methods and Reviews, Springer, Caroline J. (Cancer Research UK Centre for Cancer Therapeutics at the Institute of Cancer Research, Sutton, Surrey, UK), Humana Press, 2004) and include, for example, the Herpes Simplex Virus (HSV) thymidine kinase (TK) gene, cytosine daminase, purine nucleoside phosphorylase, and nitroreductase.

A seventh aspect of the present application provides an engineered host cell.

Further, the engineered host cell expresses the nanobody according to the first aspect of the present application, the humanized anti-CD7 nanobody according to the second aspect of the present application, the chimeric antigen receptor according to the third aspect of the present application, or the chimeric antigen receptor according to the fourth aspect of the present application;
preferably, the engineered host cell comprises the recombinant expression vector according to the sixth aspect of the present application;
preferably, the engineered host cell comprises an engineered immune cell;
more preferably, the engineered immune cell comprises a T cell, a NK cell, an iNKT cell, a CTL cell, a monocyte, a macrophage, a dendritic cell, a NKT cell or any combination thereof.

The term "host cell" as used herein refers to any type of cell. The host cell can be a eukaryotic cell, such as plant, animal, fungi, or algae, or can be a prokaryotic cell, such as bacteria or protozoa. The host cell can be a cultured cell or a primary cell, i.e., isolated directly from an organism, e.g., a human. The host cell can be an adherent cell or a suspended cell, i.e., a cell that grows in suspension. Suitable host cells are known in the art and include, for instance, DH5a E. coli cells, Chinese hamster ovarian cells, monkey VERO cells, COS cells, HEK293 cells, and the like. For purposes of amplifying or replicating the recombinant expression vector, the host cell may be a prokaryotic cell, e.g., a DH5α cell. For purposes of producing a recombinant CAR, the host cell may be a mammalian cell. The host cell may be a human cell. While the host cell can be of any cell type, can originate from any type of tissue, and can be of any developmental stage, the host cell may be a peripheral blood lymphocyte (PBL) or a peripheral blood mononuclear cell (PBMC). In a specific embodiment of the present application, the host cell is a T cell.

Further, the engineered host cell includes a population of engineered host cells.

Further, the population of engineered host cells includes host cells not expressing the nanobody according to the first aspect of the present application, the humanized anti-CD7 nanobody according to the second aspect of the present application, the chimeric antigen receptor according to the third aspect of the present application, or the chimeric antigen receptor according to the fourth aspect of the present application.

Further, the host cell is an immune cell.

Further, the immune cell comprises a T cell, a NK cell, an iNKT cell, a CTL cell, a monocyte, a macrophage, a dendritic cell, a NKT cell or any combination thereof.

An eighth aspect of the present application provides a conjugate.

Further, the conjugate comprises the nanobody according to the first aspect of the present application or the humanized anti-CD7 nanobody according to the second aspect of the present application, and a modification moiety connected to the nanobody, and the modification moiety comprises a detectable label, a therapeutic agent;
preferably, the detectable label comprises an enzyme, a radionuclide, a fluorescent dye, a luminescent substance, biotin,
preferably, the therapeutic agent comprises a cytotoxic agent or a drug with anti-tumor activity.

A ninth aspect of the present application provides a pharmaceutical composition.

Further, the pharmaceutical composition comprises the nanobody according to the first aspect of the present application, the humanized anti-CD7 nanobody according to the second aspect of the present application, the chimeric antigen receptor according to the third aspect of the present application, the chimeric antigen receptor according to the fourth aspect of the present application, the nucleic acid molecule according to the fifth aspect of the present application, the recombinant expression vector according to the sixth aspect of the present application, the engineered host cell according to the seventh aspect of the present application or the conjugate according to the eighth aspect of the present application.

Further, the pharmaceutical composition further comprises an additional pharmaceutically active agent;
preferably, the additional pharmaceutically active agent comprises an additional antibody, fusion protein or drug (such as an anti-tumor drug, such as a drug for radiotherapy or a chemotherapeutic drug).

Further, the pharmaceutical composition further comprises a pharmaceutically acceptable carrier and/or excipient.

Further, the pharmaceutically acceptable carrier and/or excipient are described in detail in Remington's Pharmaceutical Sciences (19th ed., 1995). These substances are used to help the stability of the drug or help improve the effectiveness of active ingredients as needed. The preparation that can be used in this pharmaceutical composition can be in the form of its original compound itself or optionally in the form of its pharmaceutically acceptable salt. The pharmaceutical composition thus prepared can be administered in any suitable way known to those skilled in the art as needed.

The pharmaceutically acceptable carrier and/or excipient may additionally comprise a liquid such as water, physiological saline, glycerin and ethanol. In addition, auxiliary substances such as wetting agents or emulsifying agents or pH buffering substances may be present in the composition. These carriers enable the pharmaceutical composition to be formulated as tablets, pills, dragees, capsules, liquids, gels, syrups, slurries and suspensions for ingestion by patients.

Suitable forms of administration include those suitable for parenteral administration, for example, by injection or infusion, for example, by bolus injection or continuous infusion, intravenous, inhalable or subcutaneous forms. Where the product is for injection or infusion, it may take the form of a suspension, solution or emulsion in an oily or aqueous vehicle and it may contain formulation agents such as suspending agents, preservatives, stabilizers and/or dispersants. Alternatively, the nanobody provided according to the present application may be in a dry form for reconstitution with a suitable sterile liquid prior to use. Solid forms suitable for dissolution or suspension in liquid vehicles before injection can also be prepared.

A tenth aspect of the present application provides a kit.

Further, the kit comprises the nanobody according to the first aspect of the present application, the humanized anti-CD7 nanobody according to the second aspect of the present application, the chimeric antigen receptor according to the third aspect of the present application, the chimeric antigen receptor according to the fourth aspect of the present application, the nucleic acid molecule according to the fifth aspect of the present application, the recombinant expression vector according to the sixth aspect of the present application, the engineered host cell according to the seventh aspect of the present application or the conjugate according to the eighth aspect of the present application.

An eleventh aspect of the present application provides a reagent for detecting CD7 protein or antigen fragment thereof.

Further, the reagent for detecting CD7 protein or antigen fragment thereof comprises the nanobody according to the first aspect of the present application, the humanized anti-CD7 nanobody according to the second aspect of the present application, or the conjugate according to the eighth aspect of the present application.

A twelfth aspect of the present application provides a biological preparation.

Further, the biological preparation comprises the engineered host cell according to the seventh aspect of the present application and/or the pharmaceutical composition according to the ninth aspect of the present application.

Further, the biological preparation as described in the present application, also known as a biological drug, a biological (medical) product or a biological product, is any pharmaceutical product semi-synthesized, extracted or manufactured from biological sources. They include, but not limited to, vaccines, blood or blood components, and recombinant therapeutic proteins (such as antibodies, cytokines, chemokines, and fusion proteins). The biological preparation can be composed of sugars, proteins, or nucleic acids or complex combinations of these substances.

A pharmaceutical composition or a biological preparation or a composition thereof are provided herein for use in gene therapy, immunotherapy and/or cell therapy that include one or more of the disclosed CAR, immune cell expressing the CAR, nanobody, or conjugate that specifically bind to one or more antigens disclosed herein, in a carrier (such as a pharmaceutically acceptable carrier). The pharmaceutical composition or the biological preparation or the composition thereof can be prepared in unit dosage forms for administration to a subject. The amount and timing of administration are at the discretion of the treating clinician to achieve the desired outcome. The compositions can be formulated for systemic (such as intravenus) or local (such as intra-tumor) administration. In one example, the disclosed CAR, immune cell expressing the CAR (particularly, T cell expressing the CAR), nanobody, or conjugate is formulated for parenteral administration, such as intravenous administration. The CAR, immune cell expressing the CAR (particularly, T cell expressing the CAR), conjugate or compositions thereof as disclosed herein are of use, for example, for the treatment and detection of a tumor, for example, and not by way of limitation, tumors expressing CD7. In some examples, the pharmaceutical composition or a biological preparation or a composition thereof are useful for the treatment or detection of a tumor. The CAR, immune cell expressing the CAR (particularly, T cell expressing the CAR), nanobody, conjugate or compositions thereof as disclosed herein are also of use, for example, for the detection of, for example, CD7 antigens.

The pharmaceutical composition or the biological preparation or the composition thereof for administration may comprise solution of the CAR, immune cell expressing the CAR (particularly, T cell expressing the CAR), conjugate, nanobody dissolved in a pharmaceutically acceptable carrier (such as an aqueous carrier). A variety of aqueous carriers can be used, such as buffered saline and the like. These solutions are sterile and generally free of undesirable substances. These pharmaceutical compositions or biological preparations or compositions thereof may be sterilized by conventional and well-known sterilization techniques. These pharmaceutical compositions or biological preparations or compositions thereof may contain pharmaceutically acceptable auxiliary substances as needed to approximate physiological conditions, and the pharmaceutically acceptable auxiliary substances are such as pH adjusters and buffers, toxicity adjusters, auxiliary agents, *etc.,* such as sodium acetate, sodium chloride, potassium chloride, calcium chloride, sodium lactate, *etc.* The concentration of the CAR, immune cell expressing the CAR (particularly, T cell expressing the CAR), conjugate, nanobody in these preparations can vary widely, and is selected mainly based on fluid volume, viscosity, body weight, *etc.* according to the selected specific administration mode and the needs of the subject. Practical methods of preparing such dosage forms for gene therapy, immunotherapy and/or cell therapy are known or would be apparent to those skilled in the art.

A typical composition (pharmaceutical composition or biological preparation or composition thereof) for intravenous administration includes about 0.01 to about 30 mg/kg of the nanobody according to the first aspect of the present application or the humanized anti-CD7 nanobody according to the second aspect of the present application or the engineered host cell according to the seventh aspect of the present application or the conjugate according to the eighth aspect of the present application per subject per day. Actual methods for preparing administrable compositions will be known or apparent to those skilled in the art and are described in more detail in such publications as Remington's Pharmaceutical Science, 19th ed., Mack Publishing Company, Easton, PA (1995).

The CAR, immune cell expressing the CAR (particularly, T cell expressing the CAR), nanobody or conjugate described herein may be provided in lyophilized form and rehydrated with sterile water before administration, although they are also provided in sterile solutions of known concentration. The CAR, immune cell expressing the CAR (particularly, T cell expressing the CAR), nanobody or conjugate solution is then added to an infusion bag containing 0.9% sodium chloride, USP, and in some cases administered at a dosage of from 0.5 to 15 mg/kg of body weight.

Considerable experience is available in the art in the administration of nanobody- and conjugate-related drugs; for example, antibody drugs have been marketed in the U.S. since the approval of RITUXAN^{®} in 1997. A CAR, immune cell expressing a CAR, antibodies, antigen binding fragments and conjugates thereof can be administered by slow infusion, rather than in an intravenous push or bolus. In one example, a higher loading dose is administered, with subsequent, maintenance doses being administered at a lower level. For example, an initial loading dose of 4 mg/kg antibody or antigen binding fragment (or the corresponding dose of a conjugate including the antibody or antigen binding fragment) may be infused over a period of some 90 minutes, followed by weekly maintenance doses for 4-8 weeks of 2 mg/kg infused over a 30 minutes period if the previous dose was well tolerated.

Controlled release parenteral formulations can be made as implants, oily injections, or as particulate systems. For a broad overview of protein delivery systems see, Banga, A.J., Therapeutic Peptides and Proteins: Formulation, Processing, and Delivery Systems, Technomic Publishing Company, Inc., Lancaster, PA, (1995). Particulate systems include microspheres, microparticles, microcapsules, nanocapsules, nanospheres, and nanoparticles. Microcapsules contain the therapeutic protein, such as a cytotoxin or a drug, as a central core. In microspheres, the therapeutic is dispersed throughout the particle. Particles, microspheres, and microcapsules smaller than about 1 µm are generally referred to as nanoparticles, nanospheres, and nanocapsules, respectively. Capillaries have a diameter of approximately 5 µm so that only nanoparticles are administered intravenously. Microparticles are typically around 100 µm in diameter and are administered subcutaneously or intramuscularly. See, for example, Kreuter, J., Colloidal Drug Delivery Systems, J. Kreuter, ed., Marcel Dekker, Inc., New York, NY, pp. 219- 342 (1994); and Tice & Tabibi, Treatise on Controlled Drug Delivery, A. Kydonieus, ed., Marcel Dekker, Inc. New York, NY, pp. 315-339, (1992).

Polymers can be used for ion-controlled release of the CAR, immune cell expressing the CAR (particularly, T cell expressing the CAR), nanobody, conjugate or compositions thereof as disclosed herein. Various degradable and nondegradable polymeric matrices for use in controlled drug delivery are known in the art (Langer, Accounts Chem. Res. 26:537-542, 1993). For example, the block copolymer, polaxamer 407, exists as a viscous yet mobile liquid at low temperatures but forms a semisolid gel at body temperature. It has been shown to be an effective vehicle for formulation and sustained delivery of recombinant interleukin-2 and urease (Johnston et al, Pharm. Res. 9:425-434, 1992; and Pec et al, J. Parent. Sci. Tech. 44(2):58-65, 1990). Alternatively, hydroxyapatite has been used as a microcarrier for controlled release of proteins (Ijntema et al. , Int. J. Pharm.112:215-224, 1994). In yet another aspect, liposomes are used for controlled release as well as drug targeting of the lipid-capsulated drug (Betageri et al, Liposome Drug Delivery Systems, Technomic Publishing Co., Inc., Lancaster, PA (1993)). Numerous additional systems for controlled delivery of therapeutic proteins are known (see U.S. Patent No. 5,055,303; U.S. Patent No. 5,188,837; U.S. Patent No. 4,235,871; U.S. Patent No. 4,501,728; U.S. Patent No. 4,837,028; U.S. Patent No. 4,957,735; U.S. Patent No. 5,019,369; U.S. Patent No. 5,055,303; U.S. Patent No. 5,514,670; U.S. Patent No. 5,413,797; U.S. Patent No. 5,268,164; U.S. Patent No. 5,004,697; U.S. Patent No. 4,902,505; U.S. Patent No. 5,506,206; U.S. Patent No. 5,271,961; U.S. Patent No. 5,254,342 and U.S. Patent No. 5,534,496).

A thirteenth aspect of the present application provides a method of stimulating an immune response to a population of target cells or a target tissue in a mammal.

Further, the method comprises the following steps: administering an effective amount of the engineered host cell according to the seventh aspect of the present application, the conjugate according to the eighth aspect of the present application, the pharmaceutical composition according to the ninth aspect of the present application, or the biological preparation according to the twelfth aspect of the present application to a mammal.

A fourteenth aspect of the present application provides a method of modulating an immune response in a subject.

Further, the method comprises the following steps: administering an effective amount of the engineered host cell according to the seventh aspect of the present application, the conjugate according to the eighth aspect of the present application, the pharmaceutical composition according to the ninth aspect of the present application, or the biological preparation according to the twelfth aspect of the present application to a subject.

A fifteenth aspect of the present application provides a method for producing the nanobody according to the first aspect of the present application or the humanized anti-CD7 nanobody according to the fifth aspect of the present application.

Further, the method comprises the following steps: culturing the engineered host cell according to the seventh aspect of the present application, and isolating the nanobody according to the first aspect of the present application or the humanized anti-CD7 nanobody according to the second aspect of the present application from the culture.

A sixteenth aspect of the present application provides a method for specifically inhibiting CD7 activity.

Further, the method comprises the following steps: introducing the nucleic acid molecule according to the fifth aspect of the present application into a cell of an organism, and inhibiting the CD7 activity by expressing the nanobody according to the first aspect of the present application or the humanized anti-CD7 nanobody according to the second aspect of the present application.

A seventeenth aspect of the present application provides a method of preventing and/or treating a CD7-associated disease or condition.

Further, the method comprises the following steps: administrating an effective amount of the nanobody according to the first aspect of the present application, the humanized anti-CD7 nanobody according to the second aspect of the present application, the nucleic acid molecule according to the fifth aspect of the present application, the recombinant expression vector according to the sixth aspect of the present application, the engineered host cell according to the seventh aspect of the present application, the conjugate according to the eighth aspect of the present application, the pharmaceutical composition according to the ninth aspect of the present application or the biological preparation according to the twelfth aspect of the present application to a subject with a CD7-associated disease or condition.

Further, the CD7-associated disease or condition includes a tumor expressing CD7.

Further, the tumor is a hematological tumor of T lymphocyte lineage.

Further, the tumor includes acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

Further, the CD7-associated disease or condition mentioned in the present application refers to any disease or condition associated with the expression of CD7, including but not limited to the disease types listed above, and as long as the disease or condition is associated with the expression of CD7, it is within the scope of the present application.

The terms "treat" and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the methods can provide any amount or any level of treatment or prevention of cancer in a mammal.

The treatment or prevention provided by the method can include treatment or prevention of one or more conditions or symptoms of the disease, e.g., cancer, being treated or prevented. Also, for purposes herein, "prevention" can encompass delaying the onset of the disease, or a symptom or condition thereof.

The mammal referred to herein can be any mammal, including, but not limited to, mammals of the order Rodentia, such as mice and hamsters, and mammals of the order Logomorpha, such as rabbits. The mammals may be from the order Carnivora, including Felines (cats) and Canines (dogs). The mammals may be from the order Artiodactyla, including Bovines (cows) and Swines (pigs) or of the order Perssodactyla, including Equines (horses). The mammals may be of the order Primates, Ceboids, or Simoids (monkeys) or of the order Anthropoids (humans and apes). Preferably, in a specific embodiment of the present application, the mammal is a human.

Further, the engineered host cell comprises an immune cell expressing the anti-CD7 chimeric antigen receptor (CAR) based on a single nanobody as described in the third aspect of the present application or the anti-CD7 chimeric antigen receptor based on a double nanobody described in the fourth aspect of the present application;
preferably, the immune cell comprises a T cell, a NK cell, an iNKT cell, a CTL cell, a monocyte, a macrophage, a dendritic cell, or a NKT cell;
more preferably, the engineered host cell is a CAR-T cell.

Further, the engineered host cell is administered to a subject suffering from a CD7-associated disease or condition in an amount selected from:
(1) if the subject has a body weight less than 100 kilograms (kg) and is less than 18 years old, 0.05 × 10⁶ CAR-T cells/kg of the body weight of the subject or about 0.05 × 10⁶ CAR-T cells/kg of the body weight of the subject to 5.0 × 10⁷ CAR-T cells/kg of the body weight of the subject or about 5.0 × 10⁷ CAR-T cells/kg of the body weight of the subject;
(2) If the subject has a body weight of or exceeding 100 kilograms (kg) or is older than 18 years old, 0.05 × 10⁶ CAR-T cells/kg of the body weight of the subject or about 0.05 × 10⁶ CAR-T cells/kg of the body weight of the subject to 5.0 × 10⁸ CAR-T cells/kg of the body weight of the subject or about 5.0 × 10⁸ CAR-T cells/kg of the body weight of the subject.

In some embodiments of the present application, the CAR-T cell therapy is carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, following isolation and processing are administered to the same subject.

In other embodiments of the present application, the CAR-T cell therapy is carried out by allogeneic transfer, in which the cells are isolated and/or otherwise prepared from a subject other than a subject who is to receive or who ultimately receives the cell therapy, e.g., a first subject. In such embodiments, the cells then are administered to a different subject, e.g., a second subject, of the same species. In some embodiments, the first and second subjects are genetically identical. In some embodiments, the first and second subjects are genetically similar. In some embodiments, the second subject expresses the same HLA class or supertype as the first subject.

In a specific embodiment of the present application, the CAR-T cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some embodiments, they are administered by parenteral, intrapulmonary, and intranasal, and, if desired for local treatment, intralesional administration. Parenteral infusions include intramuscular, intravenous, intraarterial, intraperitoneal, or subcutaneous administration. In some embodiments, a given dose is administered by a single bolus administration of the cells. In some embodiments, it is administered by multiple bolus administrations of the cells, for example, over a period of no more than 3 days, or by continuous infusion administration of the cells. In some embodiments, administration of the cell dose or any additional therapies, e.g., the lymphodepleting therapy, intervention therapy and/or combination therapy, is carried out via outpatient delivery.

Further, for the prevention or treatment of disease, the appropriate dosage may depend on the type of disease to be treated, the type of cells, the severity and course of the disease, whether the cells are administered for preventive or therapeutic purposes, previous therapy, the subject's clinical history and response to the cells, and the discretion of the attending physician. The pharmaceutical composition and/or CAR-T cells are in some embodiments suitably administered to the subject at one time or over a series of treatments.

In some embodiments, the CAR-T cells are administered as part of a combination treatment, such as simultaneously with or sequentially with, in any order, another therapeutic intervention, such as an antibody or engineered cell or receptor or agent, such as a cytotoxic or therapeutic agent. The cells in some embodiments are co-administered with one or more additional therapeutic agents or in connection with another therapeutic intervention, either simultaneously or sequentially in any order. In some embodiments, the additional therapeutic agent is any intervention preparation or agent known to those skilled in the art that can be used for tumor intervention therapy, and in some embodiments, the cells are administered prior to the one or more additional therapeutic agents. In some embodiments, the cells are administered after the one or more additional therapeutic agents. In some embodiments, the one or more additional agents comprise a cytokine such as IL-2, for example, to enhance persistence. In some embodiments, the method comprises administering a chemotherapeutic agent. In some embodiments, the method comprises administering a chemotherapeutic agent, e.g., a conditioning chemotherapeutic agent, for example, to reduce tumor burden prior to the administration. In some embodiments, preconditioning a subject with immunodepleting (e.g., lymphodepleting) therapy can improve the effects of cell therapy.

In some embodiments, the method and use provided herein involve the administration of all or part of CAR-T cells or pharmaceutical compositions, and the cells are engineered immune cell expressing the chimeric antigen receptor CAR according to the second aspect of the present application or the chimeric antigen receptor according to the third aspect of the present application. In some embodiments, a specific amount or number of cells or a specific amount of a pharmaceutical composition containing the specific amount or number of cells is administered to a subject. In some embodiments, one or more cell doses are administered to a subject, the one or more cell doses contain a specific amount or number of cells or a specific amount of a pharmaceutical composition containing the specific amount or number of cells. In some embodiments, a certain dose of cells is administered to a subject according to the provided methods and/or using the provided preparations or compositions. In some embodiments, the size, amount, or timing of the dose is determined based on the age of the subject. In some embodiments, the size, amount, or timing of the dose is determined based on the body weight of the subject. In some embodiments, the size, amount, or timing of the dose is determined based on the particular type of tumor in the subject.

The eighteenth aspect of the present application provides a method for detecting CD7 protein or antigen fragment thereof.

Further, the method comprises the following steps:
(1) obtaining a sample containing the CD7 protein or antigen fragment thereof;
(2) contacting the sample collected in step (1) with the nanobody according to the first aspect of the present application, the humanized anti-CD7 nanobody according to the second aspect of the present application, the conjugate according to the eighth aspect of the present application, the kit according to the tenth aspect of the present application or the reagent for detecting a CD7 protein or antigen fragment thereof according to the eleventh aspect of the present application; and
(3) detecting the presence of an antibody-antigen complex.

Further, the nanobody is a nanobody labeled with a label that can be used for detection.

Further, the label that can be used for detection comprises a fluorescent pigment, avidin, a paramagnetic atom, a radioactive isotope.

Further, the fluorescent pigment is fluorescein, rhodamine, Texas red, phycoerythrin, phycocyanin, allophycocyanin, peridinin-chlorophyll protein.

Further, the avidin is biotin, avidin derived from ovalbumin, streptavidin, avidin derived from ovi vitellus, avidin analogs.

Further, the radioactive isotope is radioactive iodine, radioactive cesium, radioactive iridium, radioactive cobalt.

A nineteenth aspect of the present application provides a method for preparing the engineered host cell according to the seventh aspect of the present application.

Further, the method comprises the following steps: introducing the recombinant expression vector according to the sixth aspect of the present application into a host cell.

Further, the method for the introducing comprises lipofection, microinjection, electroporation, DNA vectors, RNA vectors, retroviral vectors, lentiviral vectors, poxviral vectors, herpes simplex viral vectors, adenoviral vectors, adeno-associated viral vectors.

A twentieth aspect of the present application provides a method for diagnosing whether a subject has a tumor expressing CD7.

Further, the method comprises the following steps:
(1) providing a sample from a subject suspected of having a tumor expressing CD7;
(2) contacting the sample with the nanobody according to the first aspect of the present application, the humanized anti-CD7 nanobody according to the second aspect of the present application, the conjugate according to the eighth aspect of the present application, the kit according to the tenth aspect of the present application or the reagent for detecting a CD7 protein or antigen fragment thereof according to the eleventh aspect of the present application;
(3) detecting the formation of a complex comprising the nanobody and the antigen, to obtain the amount of CD7 in the sample from the subject, and comparing the amount of CD7 in the sample from the subject with the amount of CD7 in a known standard or reference sample, and determining whether the CD7 level in the sample from the subject falls within a tumor-associated CD7 level.

Further, the sample in step (1) comprises urine, blood, serum, plasma, saliva, ascites, circulating cells, circulating tumor cells, non-tissue associated cells, tissues or histological preparations.

Further, the tumor expressing CD7 is a hematological tumor of T lymphocyte lineage.

Further, the tumor includes acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

A twenty-first aspect of the present application provides use of the nanobody according to the first aspect of the present application or the humanized anti-CD7 nanobody according to the second aspect of the present application in detecting CD7 protein or antigen fragments thereof.

A twenty-second aspect of the present application provides use of the nanobody according to the first aspect of the present application or the humanized anti-CD7 nanobody according to the second aspect of the present application in preparation of a reagent or a kit for detecting CD7 protein or antigen fragment thereof.

A twenty-third aspect of the present application provides use of the nucleic acid molecule according to the fifth aspect of the present application or the recombinant expression vector according to the sixth aspect of the present application in preparation of an engineered host cell.

Further, the engineered host cell is the engineered host cell according to the seventh aspect of the present application.

A twenty-fourth aspect of the present application provides use of the nanobody according to the first aspect of the present application, the humanized anti-CD7 nanobody according to the second aspect of the present application, the chimeric antigen receptor according to the third aspect of the present application, the chimeric antigen receptor according to the fourth aspect of the present application, the nucleic acid molecule according to the fifth aspect of the present application, the recombinant expression vector according to the sixth aspect of the present application, the engineered host cell according to the seventh aspect of the present application, the conjugate according to the eighth aspect of the present application, the pharmaceutical composition according to the ninth aspect of the present application or the biological preparation according to the twelfth aspect of the present application in preparation of an anti-tumor drug.

Further, the anti-tumor drug comprises an anti-tumor immune cell therapy agent, an anti-tumor gene therapy drug.

Further, the tumor is a tumor expressing CD7.

Further, the tumor is a hematological tumor of T lymphocyte lineage.

Further, the tumor includes acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

A twenty-fifth aspect of the present application provides use of the nanobody according to the first aspect of the present application, the humanized anti-CD7 nanobody according to the second aspect of the present application, the chimeric antigen receptor according to the third aspect of the present application, the chimeric antigen receptor according to the fourth aspect of the present application, the nucleic acid molecule according to the fifth aspect of the present application, the recombinant expression vector according to the sixth aspect of the present application, the engineered host cell according to the seventh aspect of the present application, the conjugate according to the eighth aspect of the present application, the pharmaceutical composition according to the ninth aspect of the present application or the biological preparation according to the twelfth aspect of the present application in preparation of a kit for preparing an immune cell for preventing and/or treating a tumor.

Further, the tumor is a tumor expressing CD7.

Further, the tumor is a hematological tumor of T lymphocyte lineage.

Further, the tumor includes acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

A twenty-sixth aspect of the present application provides use of the nanobody according to the first aspect of the present application, the humanized anti-CD7 nanobody according to the second aspect of the present application, the chimeric antigen receptor according to the third aspect of the present application, the chimeric antigen receptor according to the fourth aspect of the present application, the nucleic acid molecule according to the fifth aspect of the present application, the recombinant expression vector according to the sixth aspect of the present application, the engineered host cell according to the seventh aspect of the present application, the conjugate according to the eighth aspect of the present application, or the pharmaceutical composition according to the ninth aspect of the present application in preparation of a biological preparation for preventing and/or treating a tumor.

Further, the tumor is a tumor expressing CD7.

Further, the tumor is a hematological tumor of T lymphocyte lineage.

Further, the tumor includes acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

A twenty-seventh aspect of the present application provides use of the engineered host cell according to the seventh aspect of the present application in prevention and/or treatment of a tumor.

Further, the tumor is a tumor expressing CD7.

Further, the tumor is a hematological tumor of T lymphocyte lineage.

Further, the tumor includes acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

A twenty-eighth aspect of the present application provides use of the conjugate according to the eighth aspect of the present application in prevention and/or treatment of a tumor.

Further, the tumor is a tumor expressing CD7.

Further, the tumor is a hematological tumor of T lymphocyte lineage.

Further, the tumor includes acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

A twenty-ninth aspect of the present application provides use of the pharmaceutical composition according to the ninth aspect of the present application in prevention and/or treatment of a tumor.

Further, the tumor is a tumor expressing CD7.

Further, the tumor is a hematological tumor of T lymphocyte lineage.

Further, the tumor includes acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

A thirtieth aspect of the present application provides use of the kit according to the tenth aspect of the present application in preparation of an immune cell for preventing and/or treating a tumor.

Further, the immune cell is the engineered host cell according to the seventh aspect of the present application.

A thirty-first aspect of the present application provides use of the reagent for detecting CD7 protein or antigen fragment thereof according to the eleventh aspect of the present application in detection of CD7 protein or antigen fragment thereof.

A thirty-second aspect of the present application provides use of the biological preparation according to the twelfth aspect of the present application in prevention and/or treatment of a tumor.

Further, the tumor is a tumor expressing CD7.

Further, the tumor is a hematological tumor of T lymphocyte lineage.

Further, the tumor includes acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

Compared with the prior art, the advantages and beneficial effects of the present application are as follows:
The present application provides an anti-CD7 nanobody, which has good affinity to CD7, and the nanobody is used as the antigen binding region of a CAR to modify the CAR and the thus modified CAR is used in CAR-T cell therapy. Compared with the conventional CAR-T cell constructed based on a monoclonal antibody (scFv), the CAR-T cell constructed based on a single nanobody or a double nanobody according to the present application has various advantages that it not only effectively avoids the defects of difficult expression and poor stability of the conventional CAR-T based on scFv, but also significantly enhances the ability of immune cells to target and recognize tumor antigens and strengthen the killing activity on tumor cells, and has broad application prospects.

### BRIEF DESCRIPTION OF THE DRAWINGS

Fig. 1 is a graph showing the SDS-PAGE result of CD7 antigen purification;
Fig. 2 is a flow chart showing immunization of alpaca;
Fig. 3 is a graph showing the result of the first round of PCR amplification during the construction of the nanobody library;
Fig. 4 is a graph showing the result of the second round of PCR amplification during the construction of the nanobody library;
Fig. 5 is a graph showing the PCR results of diversity detection of the nanobody library;
Fig. 6 is a graph showing the ratio results of the positive group to the negative group after phage panning;
Fig. 7 is a graph showing the statistical results of OD values of monoclonal screening;
Fig. 8 is a graph showing the statistical results of monoclonal screening and identification;
Fig. 9 is a schematic structural diagram of a single VHH CAR-T;
Fig. 10 is a flow chart of lentivirus packaging;
Fig. 11 is a flow chart of CAR-T cell culture;
Fig. 12 is a graph showing the representative results of flow cytometry detection of single VHH CAR-T cells;
Fig. 13 is a graph showing the statistical results of the CD7+ average MFI of single VHH CAR-T cells;
Fig. 14 is a graph showing the statistical results of killing ratio of single VHH CAR-T cells;
Fig. 15 is a graph showing the statistical results of the average MFI value of a single VHH K562 cell line;
Fig. 16 is a graph showing the results of single VHH specificity detection;
Fig. 17 is a graph showing the representative results of flow cytometry detection of single VHH CAR-T cells;
Fig. 18 is a graph showing the results of the CD7+ average MFI of single VHH CAR-T cells;
Fig. 19 is a graph showing the alignment results among the humanized sequence, DP-47, the template h-NbBcII10PGLA and the original sequence, in which the highlighted sites are mutation sites;
Fig. 20 is a schematic structural diagram of a double VHH CAR-T;
Fig. 21 is a graph showing the representative results of flow cytometry detection of double VHH CAR-T cells;
Fig. 22 is a graph showing the statistical results of the CD7+ average MFI of single VHH CAR-T cells and double VHH CAR-T cells;
Fig. 23 is a graph showing the results of proliferation curve of dVHH-D CAR-T cells;
Fig. 24 is a graph showing the killing results of dVHH-D CAR-T cells;
Fig. 25 is a graph showing the results of proliferation curve of dVHH-E CAR-T cells;
Fig. 26 is a graph showing the killing results of dVHH-E CAR-T cells.

### DETAILED DESCRIPTION OF EMBODIMENTS

The present application will be further described below with reference to specific examples, which are only used to explain the present application and cannot be understood as limiting the present application. Those of ordinary skill in the art may understand that: various changes, modifications, substitutions and variations can be made to these examples without departing from the principles and purposes of the present application, and the scope of the present application is defined by the claims and their equivalents. The experimental methods used in the following examples are conventional methods unless otherwise specified; The experimental methods used in the following examples are conventional methods unless otherwise specified; The reagents, materials, *etc.* used in the following examples can all be obtained from commercial sources unless otherwise specified.

### Example 1 Preparation of an antigen

### 1. Experimental method

RNA was extracted from T cells using an RNA extraction kit. Referring to the SuperScript^{™} II Reverse Transcriptase instruction manual and using random primers for reverse transcription, cDNA was obtained. The gene sequence of extracellular region of the antigen CD7 was obtained through PCR using cDNA as a template. The gene sequence of CD7 extracellular region was ligated into a protein expression vector for expression, and Ni column purification was performed to obtain purified CD7-His protein.

### 2. Experimental results

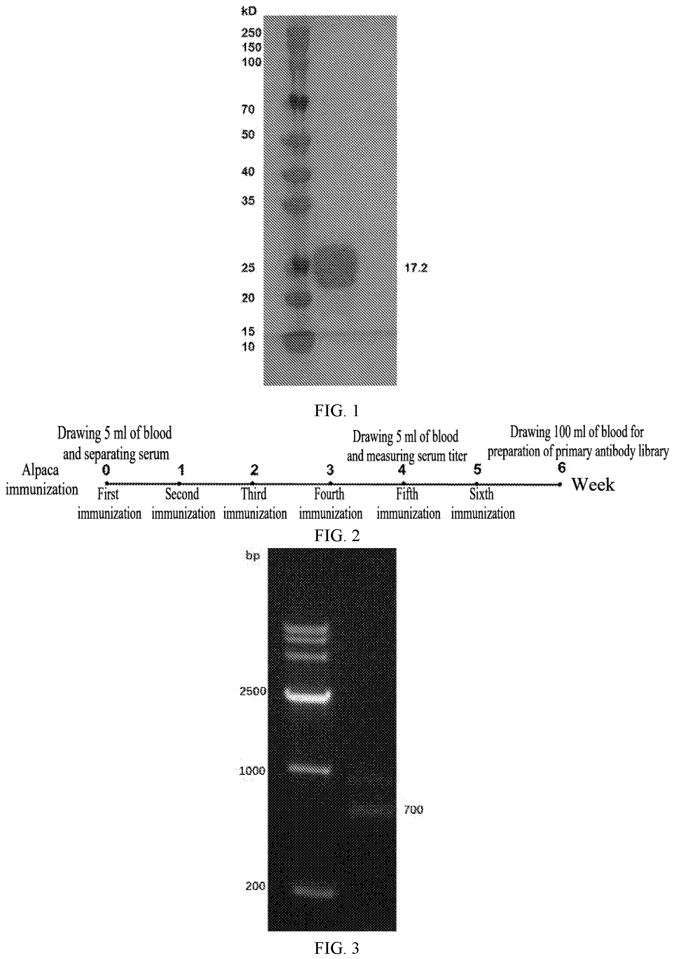

The results are shown in Fig. 1. The results show that after SDS-PAGE identification, the present application successfully prepared a CD7 antigen with a size of 17.2 kDa and a purity of > 90%, which can be used for subsequent alpaca immunization.

### Example 2 Construction of a nanobody library

1. Experimental method
   (1) The CD7-His protein purified in Example 1 was used for alpaca immunization. The specific alpaca immunization flow chart is shown in Fig. 2. Immunization was performed once a week, for a total of 6 consecutive immunizations; (3) 100 mL of peripheral blood was collected 7 days after the last immunization, peripheral blood mononuclear cells were isolated by Ficoll density gradient centrifugation, RNA was extracted, and cDNA was prepared using a reverse transcription kit; (4) VHH fragment was obtained using SOE-PCR and ligated into a pMES4 phage display vector; (5) the ligation product was electrotransformed into electroporation-competent cells TG1, and the resulting bacterial library was the constructed single domain heavy chain antibody phage display library of CD7 with library capacity of 3.37 × 10⁸; (6) after the library construction was completed, in order to determine the insertion efficiency of the library, 25 clones were randomly selected and colony PCR was performed using primers MP57 and GIII, and the PCR products were subjected to Sanger sequencing.
2. Experimental results

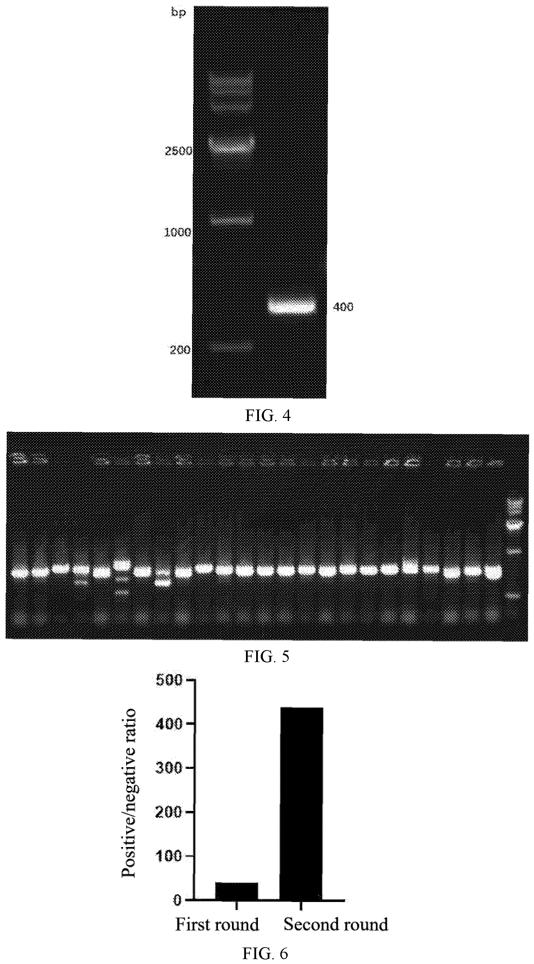

The results of the first round of PCR amplification are shown in Fig. 3. The results show that after the end of the first round of PCR, DNA fragments of approximately 700 bp are recovered. The results of the second round of PCR amplification are shown in Fig. 4. The results show that after the end of the second round of PCR, DNA fragments of approximately 400 bp are recovered. After the library construction was completed, in order to determine the insertion efficiency of the library, 25 clones were randomly selected and colony PCR was performed using primers MP57 and GIII, and the PCR products were subjected to Sanger sequencing. The results show that the insertion rate is close to 95% (see Fig. 5).

### Example 3 Enrichment screening of an Nanobody

1. Amplification of phage nanobody library
   (1) The TG1 *E. coli* nanobody library was taken and transferred to 2-YT liquid medium, cultured at 37°C and 200 rpm until the OD value was 0.5, and then helper phage VCSM13 was added to infect the cells. The mixture was mixed gently and incubated at 37°C for 30 minutes. The bacterial liquid was centrifuged to remove trace amounts of glucose, then the pellet was resuspended in 2-YT medium containing both ampicillin and kanamycin, and cultured at 37°C and 200 rpm overnight with shaking to amplify the phage displaying nanobodies. (2) The overnight culture was transferred to a 50 mL centrifuge tube and centrifuged, and the supernatant was taken, and 20% (wt/vol) PEG6000/2.5 M NaCl solution was added to precipitate the phage. Centrifugation was performed and the supernatant was discarded, the pellet was resuspended in PBS and centrifuged, and the supernatant was transferred to a new centrifuge tube, and 20% (wt/vol) PEG6000/2.5 M NaCl solution was added to reprecipitate the phage. Centrifugation was performed and the supernatant was discarded, and the pellet was resuspended in 1 mL of PBS. After centrifugation, the supernatant was transferred to a new centrifuge tube, and glycerol was added to a final concentration of 20%, and the resulting mixture was stored at -80°C. (3) To determine the titer of the phage nanobody library, the phage was diluted according to a 10-fold gradient. Phages with different dilution folds were used to infect TG1 bacteria at logarithmic growth stage, and cultured at 37°C overnight. The titer of the phage nanobody library was calculated by the number of plaque on the second day.
2. Phage enrichment and screening
   (1) The nanobodies were paned by ELISA, the recombinant CD7-His protein was coated on an ELISA plate, and incubated at 4°C overnight. (2) The ELISA plate was washed with 250 µL of PBST three times, 200 µL of blocking solution was added, and the ELISA plate was then incubated at room temperature for 2 h. (3) The corresponding phage was added to each well and incubated at room temperature for 2 h. (4) The plate was washed with 250 µL of PBST 15 times; (5) 100 µL of trypsin with a concentration of 0.25 mg/mL was added to each well, and the ELISA plate was incubated at room temperature at 700 rpm for 0.5 h. (6) the phage was eluted with AEBSF. (7) The eluted phage was used for titer determination and phage infection and amplification. (8) When the number of eluted positive phages: negative phages was ≥ 100, the panning was stopped.
3. Experimental results

The panning results are shown in Table 1 and Fig. 6. After two rounds of panning, the ratio of the positive group to the negative group reached 438 times, which has reached the standard for screening monoclones. Therefore, after two rounds of panning, the panning was stopped and the next step of screening and identification of monoclones was performed.

**Table 1 Phage panning results**

| Screening times | CD7 | Blank | Ratio |
|---|---|---|---|
| First screening | 8 × 10⁶ | 2 × 10⁵ | 40 |
| Second screening | 3.51 × 10⁸ | 8 × 10⁵ | 438 |

### Example 4 Screening and identification of positive monoclones

1. Experimental method
   (1) A single clone was selected from the TG1 *E. coli* library obtained after 2-3 rounds of screening for expanded culture, and the helper phage VCSM13 was used for infection to prepare monoclonal phage. (2) An appropriate amount of nanobody phage and K562-CD7 positive cells were incubated at room temperature for 2 h. (3) After washing the plate with PBST, HA-HRP antibody was added and incubated at room temperature for 1 h. (4) After washing the plate with PBST, 100 µL of TMB single-component chromogenic solution was added and the resulting mixture was incubated at room temperature for 30 minutes, and then 100 µL of stop solution was added. (5) A microplate reader was used to detect the absorbance at 450 nm. (6) When the ratio of the OD450 value of the sample well to the blank control was greater than 2, it was determined to be a positive clone. (7) The positive clone was subjected to bacteria liquid PCR and Sanger sequencing. (8) The Sanger sequenced monoclone was subjected to sequence alignment using the software DNAMAN and sequence-specific clones were screened.
2. Experimental results

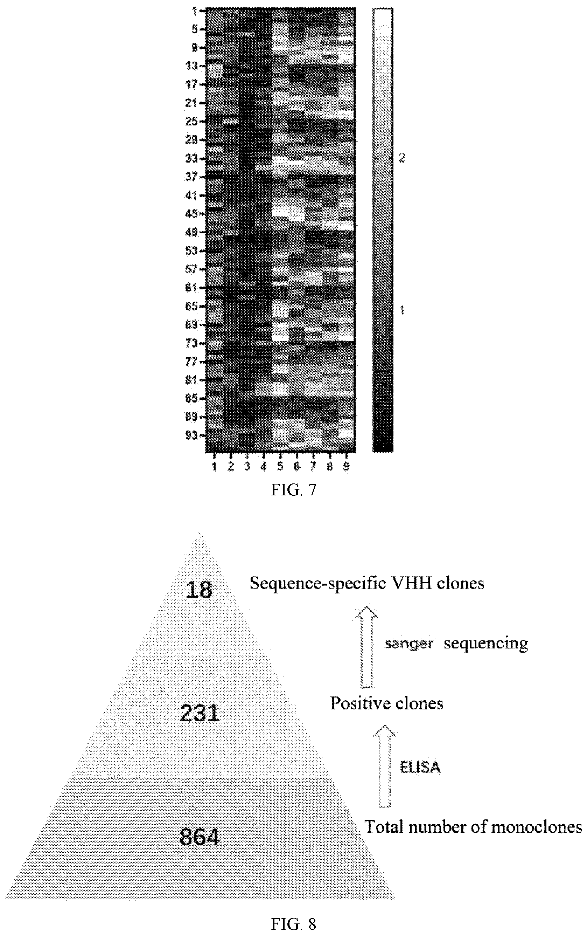

In this example, a total of 864 monoclones from 9 96-well plates were screened and the OD value results of monoclonals are shown in Fig. 7. Calculated according to the calculation principle, 231 positive clones and 17 sequence-specific clones were screened out, as shown in Fig. 8. The sequence-specific nanobodies were numbered VHH01-VHH20 (excluding VHH02, VHH05 and VHH11), respectively, VHH01, VHH03, VHH04, VHH06, VHH07, VHH08, VHH09, VHH10, VHH12, VHH13, VHH14, VHH15, VHH16, VHH17, VHH18, VHH19, and VHH20 have corresponding variable region amino acid sequences as shown in SEQ ID NO: 1, SEQ ID NO: 9, SEQ ID NO: 17, SEQ ID NO: 25, SEQ ID NO: 33, SEQ ID NO: 41, SEQ ID NO: 49, SEQ ID NO: 57, SEQ ID NO: 65, SEQ ID NO: 73, SEQ ID NO: 81, SEQ ID NO: 89, SEQ ID NO: 97, SEQ ID NO: 105, SEQ ID NO: 113, SEQ ID NO: 121, and SEQ ID NO: 129, respectively, and have corresponding variable region nucleic acid sequence as shown in SEQ ID NO: 2, SEQ ID NO: 10, SEQ ID NO: 18, SEQ ID NO: 26, SEQ ID NO: 34, SEQ ID NO: 42, SEQ ID NO: 50, SEQ ID NO: 58, SEQ ID NO: 66, SEQ ID NO: 74, SEQ ID NO: 82, SEQ ID NO: 90, SEQ ID NO: 98, SEQ ID NO: 106, SEQ ID NO: 114, SEQ ID NO: 122, SEQ ID NO: 130, respectively.

### Example 5 Preparation and in vitro functional verification of single VHH CAR-T cells

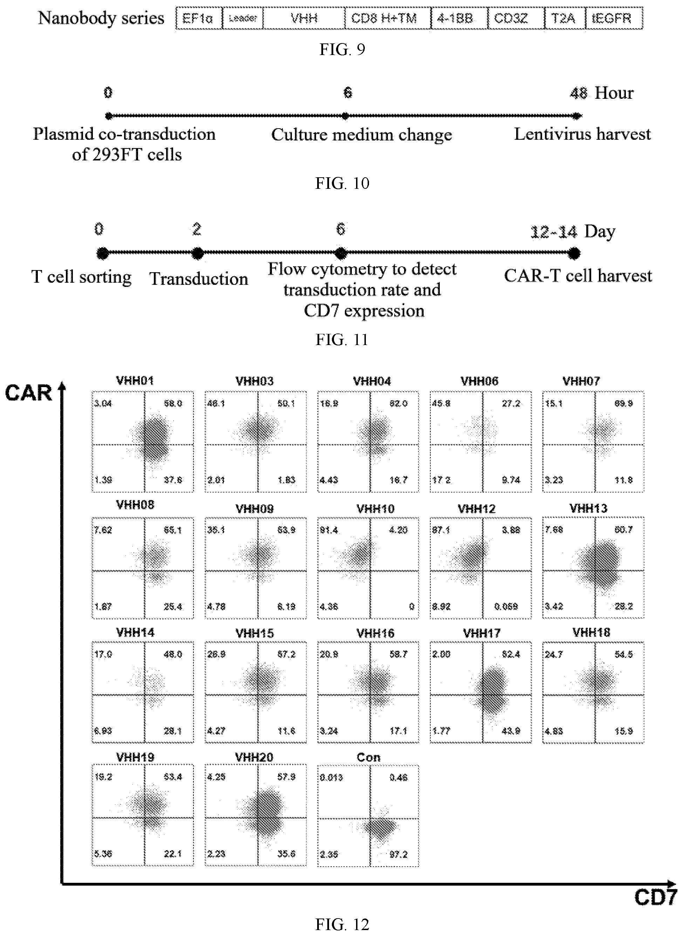

1. Construction of single VHH CAR structure
   (1) Construction of single VHH CAR structure was performed using sequence-specific clones. First, PCR was used to amplify the VHH sequence of the positive clone strain. The primers for the first round of PCR were:
      NCAR-F1: 5'-CTGCAGGAGTCTGGRGGAGG-3'
      NCAR-R1: 5'-TGAGGAGACGGTGACCTGGG-3'
      After the end of the first round of PCR, the product obtained from the first round of PCR was used as a template to perform a second round of PCR. The primers for the second round of PCR were:
      NCAR-F2: 5'-TTTCTGCTGATCCCCCAGGTGCAGCTGCAGGAGTCTGGRGGAGG-3'
      NCAR-R2: 5'-TAGGAGCCGGGGTGGGCGGCCGCGGTGCTGGGGTAGTTGAGGAGACG GTGACCTGGG-3'
   (2) The product obtained from the second round of PCR was ligated into the vector Senl-S88BZ through homologous recombination, and the vector was digested with a single Not I enzyme. At this point, a CAR structure containing a single VHH targeting CD7 was successfully constructed. A total of 17 single VHH sequences were constructed, named VHH01 to VHH20 (excluding VHH02, VHH05, and VHH11), respectively. The structural diagram of the constructed single VHH CAR-T is shown in Fig. 9, wherein EF1α is the promoter of elongation factor 1α, leader is the coding sequence of a signal peptide, VHH is the coding sequence of the anti-CD7 nanobody, CD8H+TM is CD8 hinge region and transmembrane region, 4-1BB and CD3ζ intracellular signaling regions are intracellular costimulatory domains, and an extracellular region expressing tEGFR is linked via T2A peptide, so that the expression of CAR can be detected after viral transduction.
2. Preparation of single VHH CAR-T cells

Before preparing CAR-T cells, packaging of lentivirus was firstly performed: (1) The plasmid of interest and three helper plasmids (pMD2.G, pRSV-REV, pMDLg) were co-transfected into 293FT cells under the action of PEI-Pro. (2) Packaging was performed for 6 h and then the liquid was changed. (3) Lentivirus was harvested 48 hours after packaging. (4) The harvested lentivirus stock liquid was concentrated by ultracentrifugation, and the lentiviral particles were resuspended in DMEM high-glucose medium and divided for use. The lentivirus packaging process is shown in Fig. 10;

After lentivirus packaging was completed, CAR-T cells were prepared: (1) Peripheral blood mononuclear cells (PBMC) were collected from patients or healthy donors. (2) αβT cells were sorted through CD3 magnetic beads. (3) The sorted αβT cells were cultured in TexMACS GMP medium (MACS). (4) Lentiviral transduction was performed 2 days later. (5) The culturing was continued until day 12-day 14 for CAR-T cell harvest to obtain CD7-targeting VHH NS CAR-T cells (named VHH01-VHH20, excluding VHH02, VHH05, and VHH11). CAR-T cell culture process is shown in Fig. 11. (6) Flow cytometry was performed during the culture process to determine the proportion of CAR+ cells and the average MFI value of CD7+ cells.

### 3. In vitro functional verification of single VHH CAR-T cells

In order to verify the *in vitro* biological activity of the anti-CD7 VHH CAR-T cells prepared in this example, verification using *in vitro* killing experiments was performed during the culture process: firstly, the target cells KG-1a-GFP-Luc were collected, centrifuged at 2000 rpm for 5 min, resuspend in DPBS for counting, and added to a 96-well plate at 1 × 10⁵ cells/well; then an appropriate amount of effector cells was added to target cells in E : T = 3 : 1; the effector cells and the target cells were mixed and incubated for 4 h; the corresponding luciferase substrate was added, and the luciferase value was read in the 96-well plate using an electrochemiluminescence microplate reader. Based on the value change, the killing ratio was calculated. In addition, on day 12 of culture of the 17 single VHH CAR-T cells, the 17 single VHH CAR-T cells and blank T cells were subjected to cell killing experiments with the CD7-expressing positive cell line KG-1a-GFP-Luc in the ratio of E : T = 3 : 1, respectively.

### 4. Experimental results

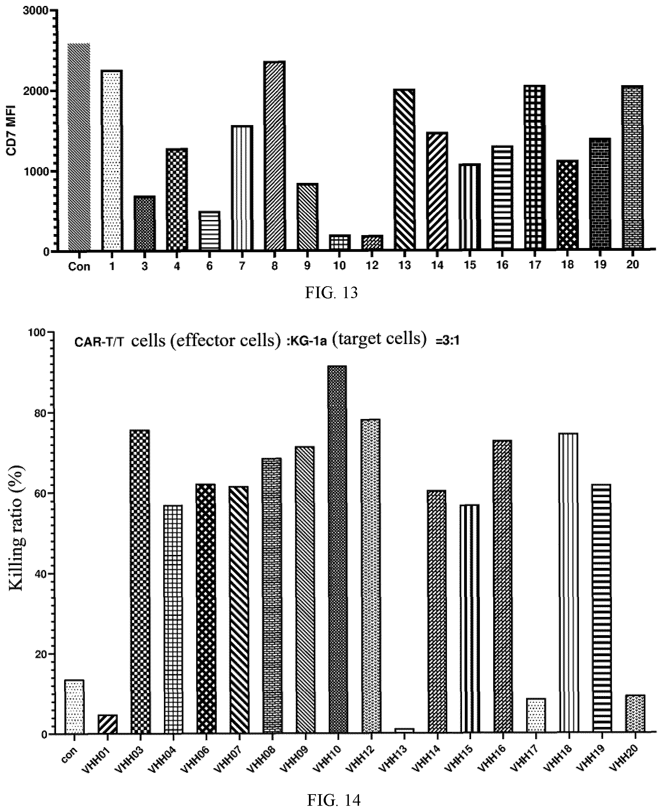

The representative results of single VHH CAR-T cells by flow cytometry and CD7+ average MFI results of single VHH CAR-T cells are shown in Fig. 12 and Fig. 13 respectively. The results show that the CAR positivity rates of VHH01 to VHH20 (excluding VHH02, VHH05 and VHH11) are: 61.04%, 96.2%, 78.9%, 83%, 85%, 72.72%, 89%, 95.6%, 90.98%, 68.38%, 65%, 84.1%, 79.6%, 54.4%, 89.2%, 72.6%, and 62.15%, respectively; the average MFI value of CD7-positive cells for blank T cells is 2588, and the average MFI value of CD7-positive cells for VHH01 to VHH20 (excluding VHH02, VHH05, and VHH11) CAR-T cells are: 2257, 694, 1281, 498, 1566, 2362, 841, 197, 190, 2010, 1473, 1077, 1300, 2054, 1117, 1391, and 2041, respectively;

The results of *in vitro* functional verification are shown in Fig. 14. The results show that the killing ratio of blank T cells is 13.65%, and the killing ratios of VHH01 to VHH20 (VHH02, VHH05 and VHH11) CAR-T cells are 4.79%, 75.7%, 57%, 62.19%, 61.63%, 68.52%, 71.47%, 91.36%, 78.18%, 1.18%, 60.44%, 56.77%, 72.87%, 8.58%, 74.52%, 61.71%, and 9.38%, respectively.

### Example 6 Detection of affinity and specificity of nanobodies

1. Affinity detection
   The VHH CAR structures corresponding to the 17 single VHHs identified through screening in Example 4 were transduced into the human myeloid leukemia cell line K562. On day 4 after transduction, flow cytometry was performed using CD7-His protein to calculate the average MFI value of positive cells. According to the difference in affinity of each VHH to CD7, the average MFI value of positive cells would be different. The higher the MFI value, the higher the affinity of the corresponding nanobody to the antigen CD7. A total of three parallel experiments were performed.
2. Specificity detection
   (1) The VHH fragments of different clone strains obtained by sequencing were cloned into the prokaryotic expression vector PET-28a-SUMO. (2) The plasmid was extracted after sequencing indicated that the sequence was correct, then transformed into *E. coli* strain BL21, and proteins were expressed under IPTG induction. (3) Crude proteins were obtained by ultrasonic lysis of bacterial cells. (4) Nanobodies were purified by ion affinity chromatography on nickel column. (5) The binding of the 17 nanobodies to K562 and K562-CD7 cell lines was detected by flow cytometry using purified nanobodies as primary antibodies and HIS-FITC antibody as secondary antibody.
3. Experimental results

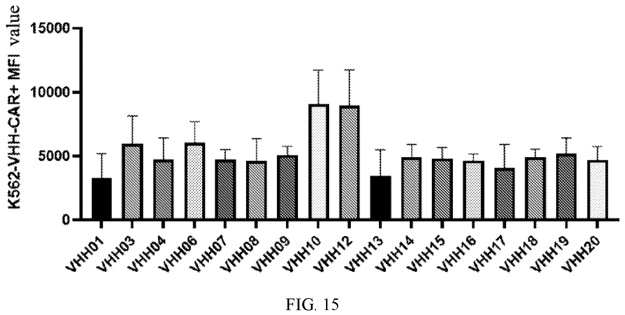

The detection results of affinity of the nanobodies are shown in Fig. 15. The results show that all the 17 single VHH structures specifically bind to CD7-His, among which VHH03, VHH06, VHHI10, and VHH12 have stronger affinity, indicating that all the 17 single VHHs obtained through screening and identification in Example 4 of the present application have good affinity to CD7.

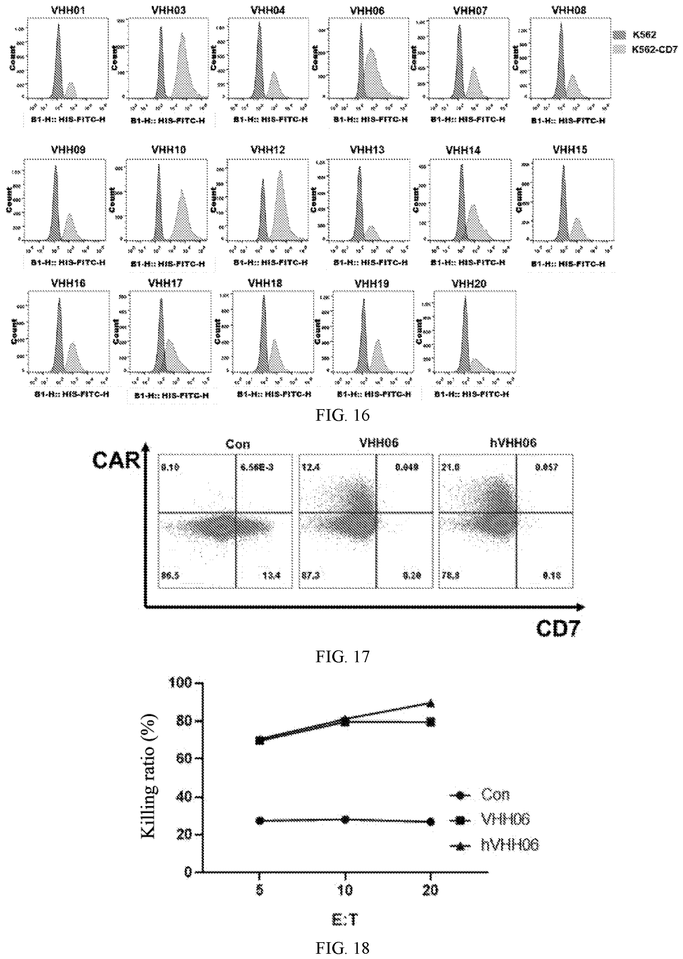

The detection results of specificity of the nanobodies are shown in Fig. 16. The results show that all the 17 VHHs can specifically bind to CD7, indicating that the 17 single VHHs obtained through screening and identification in Example 4 of the present application have good specificity.

### Example 7 Construction of humanized nanobody (hVHH06)

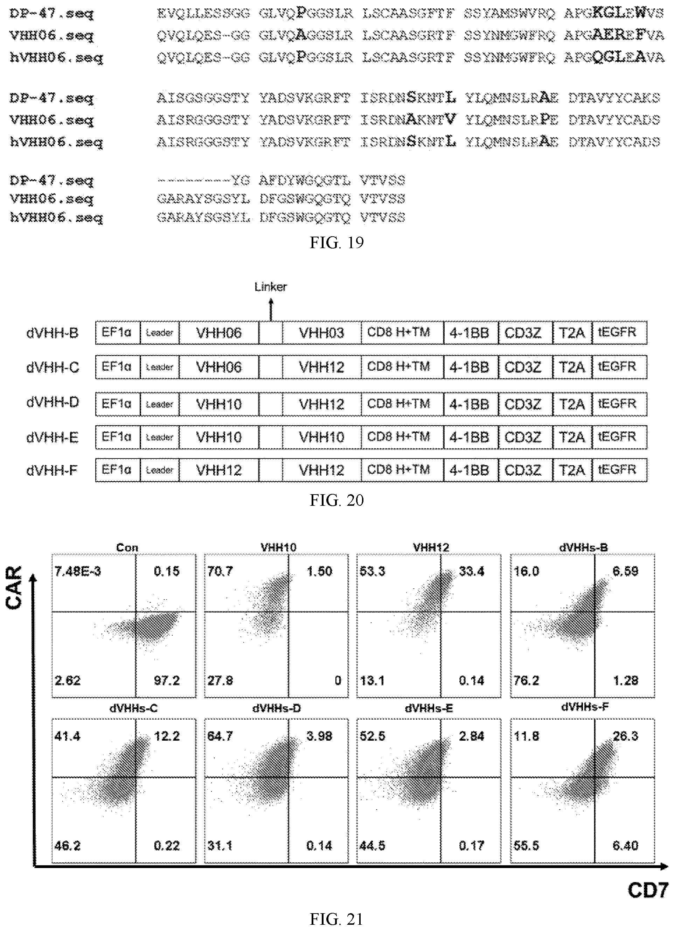

1. Experimental method
   (1) A residue at a key position in VHH06 was humanized using the universal humanization framework h-NbBcII10FGLA reported in the literature (see in details in Vincke, C., et al., General strategy to humanize a camelid single-domain antibody and identification of a universal humanized nanobody scaffold. J Biol Chem, 2009. 284(5): p. 3273-3284) as a reference via alignment with DP-47. The engineered nanobody was named hVHH06, and the sequence alignment of three structures (between the humanized sequence and the DP-47, template h-NbBcII10PGLA and original sequence) is shown in Fig. 19. The amino acid sequences of CDR1, CDR2 and CDR3 of the hVHH06 are as shown in SEQ ID NO: 139, SEQ ID NO: 141 and SEQ ID NO: 143, respectively; the nucleotide sequences of CDR1, CDR2 and CDR3 are as shown in SEQ ID NO: 140, SEQ ID NO: 142 and SEQ ID NO: 144, respectively; and the amino acid sequence of the hVHH06 is as shown in SEQ ID NO: 137 and the nucleotide sequence of the hVHH06is as shown in SEQ ID NO: 138. (2) hVHH06 and VHH06 were subjected to lentivirus packaging simultaneously according to the aforementioned method, and CAR-T cells were prepared. (3) Detection was performed by flow cytometry on the day 6 of CAR-T culture. (4) On the day 10 of CAR-T culture, a killing experiment was performed using KG-1a-GFP-Luc as the target cells at effector cell:target cell ratio of E : T = 5 : 1, 10 : 1, 20 : 1 follow the aforementioned method.
2. Experimental results
   The detection results of the flow cytometry are shown in Fig. 17. The results show that the transduction rates of VHH06 and hVHH06 are 12.4% and 21% respectively, and the CD7 positive rates are 0.249% and 0.157% respectively. The results of the killing experiment are shown in Fig. 18. The results show that when E : T = 5 : 1, 10 : 1, 20 : 1, the average killing value of blank T cells against KG-1a-GFP-Luc cells was 27.40%, 28.00%, and 26.80% respectively; the average killing value of VHH06 against KG-1a-GFP-Luc cells were 69.90%, 79.80%, and 79.70% respectively; and the average killing value of hVHH06 against KG-1a-GFP-Luc cells were 70.80%, 81.20%, and 89.60% respectively. From the above results, it can be seen that humanization has no effect on the traits of the antibodies, indicating that VHH06 was successfully humanized in this example.

### Example 8 Preparation of double VHH CAR-T cells

1. Construction of double VHH CAR structure
   (1) The VHHs (VHH03, VHH06, VHH10, and VHH12) with good functions screened in Example 6 were used to construct the plasmid of interest for the double VHH CAR structure. The structural schematic diagram is shown in Fig. 20. First, PCR was used to amplify the VHH sequence of the positive clone strain. The primers for the first round of PCR were:
      dNCAR-F1: 5'-CAGGTGCAGCTGCAGGAG-3'
      dNCAR-R1: 5'-TGAGGAGACGGTGACCTGG-3'
      After the end of the first round of PCR, the product obtained from the first round of PCR was used as a template to perform a second round of PCR. The primers for the second round of PCR were:
      dNCAR-F2 : 
5'-CCAGGTCACCGTCTCCTCAGGAGGAGGAGGATCCGGAGGAGGAGGATCTGGCGGCGGCGGCAGTG
GCGGCGGCGGCTCCGGCGGCGGCGGCTCTCAGGTGCAGCTGCAGGAG-3'
      dNCAR-F1 : 5'-TAGGAGCCGGGGTGGGGGGGCCGCGGTGCTGGGGTATTGAGGAGACGGTGACCTGG-3'
      Then, the product obtained from the second round of PCR was ligated into the vector VHH-XX (XX represents the numbering of the single VHH CAR structure targeting CD7) through homologous recombination, and the vector was digested with a single Not I enzyme. Among them, the double VHH structures constructed with VHH03 and VHH12 respectively using VHH-06 as the vector were named dVHH-B and dVHH-C respectively. The double VHH structures constructed with VHH12 and VHH10 respectively using VHH-10 as the vector were named dVHH-D and dVHH-E respectively. The double VHH structure constructed with VHH12 using VHH12 as the vector was named dVHH-F.
2. Preparation of double VHH CAR-T cells
   The preparation process of double VHH CAR-T cells was consistent with the preparation process of single VHH CAR-T cells in Example 5. The lentivirus packaging process is shown in Fig. 10, and the culture process is shown in Fig. 11. CAR-T cells, VHH10 CAR-T cells and VHH12 CAR-T cells prepared by using 5 double VHH structures and transduction of T cells were cultured for 6 days and then tested by flow cytometry.
3. Experimental results

In this example, a total of 5 double VHH structures were constructed, named dVHH-B to dVHH-F (VHH-06 + VHH-03, VHH-06 + VHH-12, VHH-10 + VHH-12, VHH-10 + VHH-10, VHH-12 + VHH-12), respectively, and the structures are shown in Fig. 20, wherein EF1α is the promoter of elongation factor 1α, leader is the coding sequence of a signal peptide, VHH is the coding sequence of the anti-CD7 nanobody, CD8H+TM is CD8 hinge region and transmembrane region, 4-1BB and CD3ζ intracellular signaling regions are intracellular costimulatory domains, and an extracellular region expressing tEGFR is linked via T2A peptide, so that the expression of CAR can be detected after viral transduction.

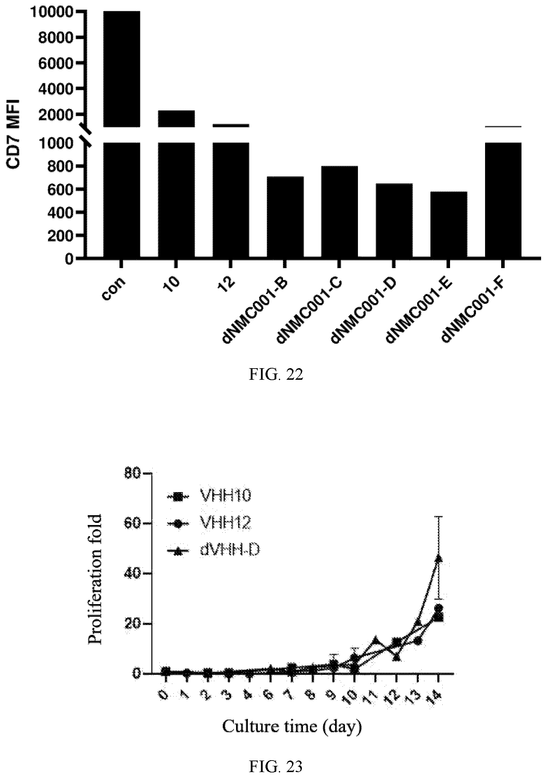

The results corresponding to the proportion of CAR+ cells and CD7 MFI values are shown in Fig. 21 and Fig. 22 respectively. The results show that the CAR positive rates of dVHH-B to dVHH-F are: 22.59%, 53.6%, 68.68%, and 55.34% respectively, the positive rate of VHH10 is 72.2%, and the positive rate of VHH12 is 86.7%; the average MFI value of CD7-positive cells for blank T cells is 10109, and the average MFI value of CD7-positive cells for CAR-T cells of dVHH-B to dVHH-F are: 708, 797, 648, 577, and 1057 respectively, the average MFI value of CD7-positive cells for CAR-T cells of VHH10 is 2302, and the average MFI value of CD7-positive cells for CAR-T cells of VHH12 is 1238.

### Example 9 Culture and in vitro functional experimental verification of double VHH CAR-T cells (dVHH-D)

### 1. Preparation of dVHH-D double VHH CAR-T cells

The dVHH-D, VHH10, and VHH12 structures were cultured *in vitro* using the CAR-T cell preparation method described in Example 8, and the cell proliferation folds during the culture process were counted.

### 2. In vitro functional experimental verification of dVHH-D double VHH CAR-T cells

To compare the *in vitro* functions of dVHH-D and single VHH CAR-T, on the day 12 of culture of dVHH-D, VHH10, and VHH12 CAR-T cells, CAR-T cells and blank T cells were subjected to cell killing experiments with the CD7-expressing positive cell line CCRF-CEM (leukemia T lymphoma cells) in the ratio of E : T = 2 : 1, respectively. In the experiments, firstly, target cells were collected, centrifuged at 2000 rpm for 5 min, resuspended in DPBS for counting, stained with CFSE, and added to a 96-well plate at an amount of 1E5/well; then, according to the different ratios of effector cells to target cells (E : T = 0.5 : 1, 1 : 1, 2 : 1), an appropriate amount of the effector cells was added to the target cells, mixed and incubated for 4 h, and the cell killing ratio was detected by flow cytometry.

### 3. Experimental results

The dVHH-D proliferation curve is shown in Fig. 23. The results show that on the day 14 of proliferation, the average proliferation fold of dVHH-D is 46.35, the average proliferation fold of VHH-12 is 26.3, and the average proliferation fold of VHH-10 is 22.75. It can be seen that the proliferation fold of dVHH-D is significantly better than that of single VHH CAR-T.

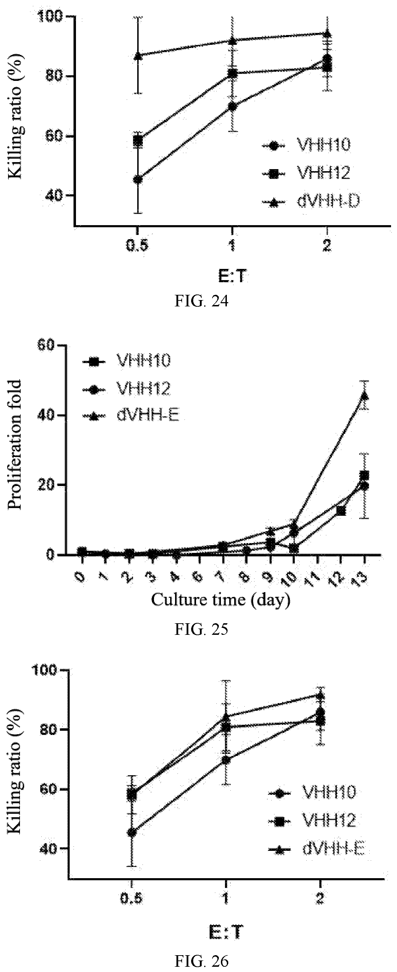

The results of the *in vitro* functional verification of dVHH-D are shown in Fig. 24. The results show that when the killing ratio is 0.5 : 1, 1 : 1, 2 : 1, the average killing value of dVHH-D against CCRF-CEM is 86.925%, 92.115%, and 94.465% respectively, the average killing value of VHH10 against CCRF-CEM is 45.55%, 69.95%, and 85.85% respectively, and the killing value of VHH12 against CCRF-CEM is 58.65%, 80.9%, and 82.95% respectively. It can be seen that dVHH-D has a higher lethality to CCRF-CEM.

### Example 10 Culture and in vitro functional experimental verification of double VHH CAR-T cells (dVHH-E)

### 1. Preparation of dVHH-E double VHH CAR-T cells

The dVHH-E, VHH10, and VHH12 structures were cultured *in vitro* using the CAR-T cell preparation method described in Example 8, and the cell proliferation folds during the culture process were counted.

### 2. In vitro functional experimental verification of dVHH-E double VHH CAR-T cells

*In vitro* functional experimental verification of dVHH-E double VHH CAR-T was performed using the method described in Example 9.

### 3. Experimental results

The dVHH-E proliferation curve is shown in Fig. 25. The results show that on the day 13 of proliferation, the average proliferation fold of dVHH-E is 45.875, the average proliferation fold of VHH-12 is 19.775, and the average proliferation fold of VHH-10 is 21.36. It can be seen that the proliferation fold of dVHH-E is significantly better than that of single VHH CAR-T.

The results of the *in vitro* functional verification of dVHH-E are shown in Fig. 26. The results show that when the killing ratio is 0.5 : 1, 1 : 1, 2 : 1, the average killing value of dVHH-E against CCRF-CEM is 58.15%, 84.385%, and 91.775% respectively, the average killing value of VHH10 against CCRF-CEM is 45.55%, 69.95%, and 85.85% respectively, and the killing value of VHH12 against CCRF-CEM is 58.65%, 80.9%, and 82.95% respectively. It can be seen that dVHH-E shows relatively higher lethality to CCRF-CEM.

### Example 11 Determination of affinities of antibodies (dVHH-D, dVHH-E, VHH10, and VHH12) by SPR method

### 1. Experimental method

In this example, the affinities of the antibodies were measured using the SPR method (surface plasmon resonance method) by immobilizing the CD7-His protein prepared in Example 1 on the CM5 chip through amino coupling method, using the antibodies (dVHH-D, dVHH-E, VHH10, and VHH12) as analytes for the experiments to detect the affinity of each antibody to the CD7 antigen.

### 2. Experimental results

The results show that the affinity constant between dVHH-D and CD7-His protein is 3.35E-09 M, the affinity constant between dVHH-E and CD7-His protein is 4.51E-09 M, the affinity constant between VHH10 and CD7-His protein is 9.99E-08 M and the affinity constant between VHH12 and CD7-His protein is 1.34E-09 M (see Table 2). The above results further show that dVHH-D, dVHH-E, VHH10, and VHH12 all can specifically bind to the CD7 antigen and have strong affinity.

**Table 2 Result summary of affinities of the antibodies determined by SPR method**

| Ligand | Analyte | Binding rate constant ka (1/Ms) | Dissociation rate number kd (1/s) | Affinity KD (M) | Chi² (RU²) | U-value |
|---|---|---|---|---|---|---|
| CD7-His protein | dVHH-D | 2.25E+05 | 7.54E-04 | 3.35E-09 | 0.0644 | 2 |
| | dVHH-E | 1.23E+05 | 5.57E-04 | 4.51E-09 | 0.0747 | 2 |
| | VHH10 | 1.64E+05 | 0.01643 | 9.99E-08 | 0.5200 | 12 |
| | VHH12 | 7.42E+04 | 9.95E-05 | 1.34E-09 | 0.0480 | 15 |

The description of the above examples is only for understanding the method and the core idea of the present application. Several improvements and modifications can also be made to the present application, and these improvements and modifications will also fall within the scope of the claims of the present application.

## Claims

1. An anti-CD7 nanobody, wherein the nanobody is any one of VHH01, VHH03, VHH04, VHH06, VHH07, VHH08, VHH09, VHH10, VHH12, VHH13, VHH14, VHH15, VHH16, VHH17, VHH18, VHH19 or VHH20;
the amino acid sequences of CDR1, CDR2 and CDR3 of the VHH01 are as shown in SEQ ID NO: 3, SEQ ID NO: 5 and SEQ ID NO: 7 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 3, SEQ ID NO: 5 and SEQ ID NO: 7 respectively;
the amino acid sequences of CDR1, CDR2 and CDR3 of the VHH03 are as shown in SEQ ID NO: 11, SEQ ID NO: 13 and SEQ ID NO: 15 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 11, SEQ ID NO: 13 and SEQ ID NO: 15 respectively;
the amino acid sequences of CDR1, CDR2 and CDR3 of the VHH04 are as shown in SEQ ID NO: 19, SEQ ID NO: 21 and SEQ ID NO: 23 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 19, SEQ ID NO: 21 and SEQ ID NO: 23 respectively;
the amino acid sequences of CDR1, CDR2 and CDR3 of the VHH06 are as shown in SEQ ID NO: 27, SEQ ID NO: 29 and SEQ ID NO: 31 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 27, SEQ ID NO: 29 and SEQ ID NO: 31 respectively;
the amino acid sequences of CDR1, CDR2 and CDR3 of the VHH07 are as shown in SEQ ID NO: 35, SEQ ID NO: 37 and SEQ ID NO: 39 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 35, SEQ ID NO: 37 and SEQ ID NO: 39 respectively;
the amino acid sequences of CDR1, CDR2 and CDR3 of the VHH08 are as shown in SEQ ID NO: 43, SEQ ID NO: 45 and SEQ ID NO: 47 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 43, SEQ ID NO: 45 and SEQ ID NO: 47 respectively;
the amino acid sequences of CDR1, CDR2 and CDR3 of the VHH09 are as shown in SEQ ID NO: 51, SEQ ID NO: 53 and SEQ ID NO: 55 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 51, SEQ ID NO: 53 and SEQ ID NO: 55 respectively;
the amino acid sequences of CDR1, CDR2 and CDR3 of the VHH10 are as shown in SEQ ID NO: 59, SEQ ID NO: 61 and SEQ ID NO: 63 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 59, SEQ ID NO: 61 and SEQ ID NO: 63 respectively;
the amino acid sequences of CDR1, CDR2 and CDR3 of the VHH12 are as shown in SEQ ID NO: 67, SEQ ID NO: 69 and SEQ ID NO: 71 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 67, SEQ ID NO: 69 and SEQ ID NO: 71 respectively;
the amino acid sequences of CDR1, CDR2 and CDR3 of the VHH113 are as shown in SEQ ID NO: 75, SEQ ID NO: 77 and SEQ ID NO: 79 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 75, SEQ ID NO: 77 and SEQ ID NO: 79 respectively;
the amino acid sequences of CDR1, CDR2 and CDR3 of the VHH14 are as shown in SEQ ID NO: 83, SEQ ID NO: 85 and SEQ ID NO: 87 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 83, SEQ ID NO: 85 and SEQ ID NO: 87 respectively;
the amino acid sequences of CDR1, CDR2 and CDR3 of the VHH15 are as shown in SEQ ID NO: 91, SEQ ID NO: 93 and SEQ ID NO: 95 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 91, SEQ ID NO: 93 and SEQ ID NO: 95 respectively;
the amino acid sequences of CDR1, CDR2 and CDR3 of the VHH16 are as shown in SEQ ID NO: 99, SEQ ID NO: 101 and SEQ ID NO: 103 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 99, SEQ ID NO: 101 and SEQ ID NO: 103 respectively;
the amino acid sequences of CDR1, CDR2 and CDR3 of the VHH17 are as shown in SEQ ID NO: 107, SEQ ID NO: 109 and SEQ ID NO: 111 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 107, SEQ ID NO: 109 and SEQ ID NO: 111 respectively;
the amino acid sequences of CDR1, CDR2 and CDR3 of the VHH18 are as shown in SEQ ID NO: 115, SEQ ID NO: 117 and SEQ ID NO: 119 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 115, SEQ ID NO: 117 and SEQ ID NO: 119 respectively;
the amino acid sequences of CDR1, CDR2 and CDR3 of the VHH19 are as shown in SEQ ID NO: 123, SEQ ID NO: 125 and SEQ ID NO: 127 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 123, SEQ ID NO: 125 and SEQ ID NO: 127 respectively;
and the amino acid sequences of CDR1, CDR2 and CDR3 of the VHH20 are as shown in SEQ ID NO: 131, SEQ ID NO: 133 and SEQ ID NO: 135 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 131, SEQ ID NO: 133 and SEQ ID NO: 135 respectively.

2. The nanobody according to claim 1, wherein the nucleotide sequences of CDR1, CDR2 and CDR3 of the VHH01 are as shown in SEQ ID NO: 4, SEQ ID NO: 6 and SEQ ID NO: 8 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 4, SEQ ID NO: 6 and SEQ ID NO: 8 respectively;
the nucleotide sequences of CDR1, CDR2 and CDR3 of the VHH03 are as shown in SEQ ID NO: 12, SEQ ID NO: 14 and SEQ ID NO: 16 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 12, SEQ ID NO: 14 and SEQ ID NO: 16 respectively;
the nucleotide sequences of CDR1, CDR2 and CDR3 of the VHH04 are as shown in SEQ ID NO: 20, SEQ ID NO: 22 and SEQ ID NO: 24 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 20, SEQ ID NO: 22 and SEQ ID NO: 24 respectively;
the nucleotide sequences of CDR1, CDR2 and CDR3 of the VHH06 are as shown in SEQ ID NO: 28, SEQ ID NO: 30 and SEQ ID NO: 32 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 28, SEQ ID NO: 30 and SEQ ID NO: 32 respectively;
the nucleotide sequences of CDR1, CDR2 and CDR3 of the VHH07 are as shown in SEQ ID NO: 36, SEQ ID NO: 38 and SEQ ID NO: 40 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 36, SEQ ID NO: 38 and SEQ ID NO: 40 respectively;
the nucleotide sequences of CDR1, CDR2 and CDR3 of the VHH08 are as shown in SEQ ID NO: 44, SEQ ID NO: 46 and SEQ ID NO: 48 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 44, SEQ ID NO: 46 and SEQ ID NO: 48 respectively;
the nucleotide sequences of CDR1, CDR2 and CDR3 of the VHH09 are as shown in SEQ ID NO: 52, SEQ ID NO: 54 and SEQ ID NO: 56 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 52, SEQ ID NO: 54 and SEQ ID NO: 56 respectively;
the nucleotide sequences of CDR1, CDR2 and CDR3 of the VHH10 are as shown in SEQ ID NO: 60, SEQ ID NO: 62 and SEQ ID NO: 64 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 60, SEQ ID NO: 62 and SEQ ID NO: 64 respectively;
the nucleotide sequences of CDR1, CDR2 and CDR3 of the VHH12 are as shown in SEQ ID NO: 68, SEQ ID NO: 70 and SEQ ID NO: 72 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 68, SEQ ID NO: 70 and SEQ ID NO: 72 respectively;
the nucleotide sequences of CDR1, CDR2 and CDR3 of the VHH13 are as shown in SEQ ID NO: 76, SEQ ID NO: 78 and SEQ ID NO: 80 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 76, SEQ ID NO: 78 and SEQ ID NO: 80 respectively;
the nucleotide sequences of CDR1, CDR2 and CDR3 of the VHH14 are as shown in SEQ ID NO: 84, SEQ ID NO: 86 and SEQ ID NO: 88 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 84, SEQ ID NO: 86 and SEQ ID NO: 88 respectively;
the nucleotide sequences of CDR1, CDR2 and CDR3 of the VHH15 are as shown in SEQ ID NO: 92, SEQ ID NO: 94 and SEQ ID NO: 96 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 92, SEQ ID NO: 94 and SEQ ID NO: 96 respectively;
the nucleotide sequences of CDR1, CDR2 and CDR3 of the VHH16 are as shown in SEQ ID NO: 100, SEQ ID NO: 102 and SEQ ID NO: 104 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 100, SEQ ID NO: 102 and SEQ ID NO: 104 respectively;
the nucleotide sequences of CDR1, CDR2 and CDR3 of the VHH17 are as shown in SEQ ID NO: 108, SEQ ID NO: 110 and SEQ ID NO: 112 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 108, SEQ ID NO: 110 and SEQ ID NO: 112 respectively;
the nucleotide sequences of CDR1, CDR2 and CDR3 of the VHH18 are as shown in SEQ ID NO: 116, SEQ ID NO: 118 and SEQ ID NO: 120 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 116, SEQ ID NO: 118 and SEQ ID NO: 120 respectively;
the nucleotide sequences of CDR1, CDR2 and CDR3 of the VHH19 are as shown in SEQ ID NO: 124, SEQ ID NO: 126 and SEQ ID NO: 128 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 124, SEQ ID NO: 126 and SEQ ID NO: 128 respectively;
and the nucleotide sequences of CDR1, CDR2 and CDR3 of the VHH20 are as shown in SEQ ID NO: 132, SEQ ID NO: 134 and SEQ ID NO: 136 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 132, SEQ ID NO: 134 and SEQ ID NO: 136 respectively.

3. The nanobody according to claim 2, wherein the amino acid sequences of the VHH01, VHH03, VHH04, VHH06, VHH07, VHH08, VHH09, VHH10, VHH12, VHH13, VHH14, VHH15, VHH16, VHH17, VHH18, VHH19, and VHH20 are as shown in SEQ ID NO: 1, SEQ ID NO: 9, SEQ ID NO: 17, SEQ ID NO: 25, SEQ ID NO: 33, SEQ ID NO: 41, SEQ ID NO: 49, SEQ ID NO: 57, SEQ ID NO: 65, SEQ ID NO: 73, SEQ ID NO: 81, SEQ ID NO: 89, SEQ ID NO: 97, SEQ ID NO: 105, SEQ ID NO: 113, SEQ ID NO: 121, and SEQ ID NO: 129 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 1, SEQ ID NO: 9, SEQ ID NO: 17, SEQ ID NO: 25, SEQ ID NO: 33, SEQ ID NO: 41, SEQ ID NO: 49, SEQ ID NO: 57, SEQ ID NO: 65, SEQ ID NO: 73, SEQ ID NO: 81, SEQ ID NO: 89, SEQ ID NO: 97, SEQ ID NO: 105, SEQ ID NO: 113, SEQ ID NO: 121, and SEQ ID NO: 129 respectively.

4. The nanobody according to claim 3, wherein the nucleotide sequences of the VHH01, VHH03, VHH04, VHH06, VHH07, VHH08, VHH09, VHH10, VHH12, VHH13, VHH14, VHH15, VHH16, VHH17, VHH18, VHH19, and VHH20 are as shown in SEQ ID NO: 2, SEQ ID NO: 10, SEQ ID NO: 18, SEQ ID NO: 26, SEQ ID NO: 34, SEQ ID NO: 42, SEQ ID NO: 50, SEQ ID NO: 58, SEQ ID NO: 66, SEQ ID NO: 74, SEQ ID NO: 82, SEQ ID NO: 90, SEQ ID NO: 98, SEQ ID NO: 106, SEQ ID NO: 114, SEQ ID NO: 122, and SEQ ID NO: 130 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 2, SEQ ID NO: 10, SEQ ID NO: 18, SEQ ID NO: 26, SEQ ID NO: 34, SEQ ID NO: 42, SEQ ID NO: 50, SEQ ID NO: 58, SEQ ID NO: 66, SEQ ID NO: 74, SEQ ID NO: 82, SEQ ID NO: 90, SEQ ID NO: 98, SEQ ID NO: 106, SEQ ID NO: 114, SEQ ID NO: 122, and SEQ ID NO: 130 respectively.

5. A humanized anti-CD7 nanobody, wherein the humanized anti-CD7 nanobody is obtained by humanizing a residue at a key position in the nanobody according to claim 1 using the universal humanization framework h-NbBcII10FGLA as a reference via alignment with DP-47.

6. The humanized anti-CD7 nanobody according to claim 5, wherein the humanized anti-CD7 nanobody is any one of hVHH01, hVHH03, hVHH04, hVHH06, hVHH07, hVHH08, hVHH09, hVHH10, hVHH12, hVHH13, hVHH14, hVHH15, hVHH16, hVHH17, hVHH18, hVHH19 or hVHH20.

7. The humanized anti-CD7 nanobody according to claim 6, wherein the amino acid sequences of CDR1, CDR2 and CDR3 of the hVHH06 are as shown in SEQ ID NO: 139, SEQ ID NO: 141 and SEQ ID NO: 143 respectively or are amino acid sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 139, SEQ ID NO: 141 and SEQ ID NO: 143 respectively.

8. The humanized anti-CD7 nanobody according to claim 7, wherein the nucleotide sequences of CDR1, CDR2 and CDR3 of the hVHH06 are as shown in SEQ ID NO: 140, SEQ ID NO: 142 and SEQ ID NO: 144 respectively or are nucleotide sequences at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 140, SEQ ID NO: 142 and SEQ ID NO: 144 respectively.

9. The humanized anti-CD7 nanobody according to claim 7, wherein the amino acid sequence of the hVHH06 is as shown in SEQ ID NO: 137 or is an amino acid sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 137.

10. The humanized anti-CD7 nanobody according to claim 9, wherein the nucleotide sequence of the hVHH06 is as shown in SEQ ID NO: 138 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 138.

11. A chimeric antigen receptor based on a single nanobody, wherein the chimeric antigen receptor comprises any one of the nanobody according to claim 1 or the humanized anti-CD7 nanobody according to claim 5.

12. The chimeric antigen receptor according to claim 11, wherein the chimeric antigen receptor further comprises a transmembrane domain.

13. The chimeric antigen receptor according to claim 12, wherein the transmembrane domain comprises a transmembrane domain of the following molecules: CD8α, CD28, IgG1, IgG4, 4-1BB, PD-1, CD34, OX40, CD3ε, IL-2 receptor, IL-7 receptor, IL-11 receptor.

14. The chimeric antigen receptor according to claim 13, wherein the transmembrane domain is a CD8α transmembrane domain.

15. The chimeric antigen receptor according to claim 12, wherein the chimeric antigen receptor further comprises an intracellular signaling domain.

16. The chimeric antigen receptor according to claim 15, wherein the intracellular signaling domain comprises an intracellular signaling domain of the following molecules: CD3ζ, FcRγ, FcRβ, CD3γ, CD3δ, CD3ε, TCRζ, CD4, CD5, CD8, CD21, CD22, CD79a, CD79b, CD278, FcεRI, DAP10, DAP12, CD66d.

17. The chimeric antigen receptor according to claim 16, wherein the intracellular signaling domain is a CD3ζ intracellular signaling domain.

18. The chimeric antigen receptor according to claim 15, wherein the chimeric antigen receptor further comprises a hinge region.

19. The chimeric antigen receptor according to claim 18, wherein the hinge region comprises a hinge region of the following molecules: CD8α, CD28, IgG1, IgG4, 4-1BB, PD-1, CD34, OX40, CD3ε, IL-2 receptor, IL-7 receptor, IL-11 receptor.

20. The chimeric antigen receptor according to claim 19, wherein the hinge region is a CD8α hinge region.

21. The chimeric antigen receptor according to claim 18, wherein the chimeric antigen receptor further comprises a signal peptide.

22. The chimeric antigen receptor according to claim 21, wherein the signal peptide comprises a signal peptide of the following molecules: alpha and beta chains of T cell receptor, CD3ζ, CD3ε, CD4, CD5, CD8, CD9, CD28, CD16, CD22, CD33, CD37, CD45, CD64, CD80, CD86, CD134, CD137, CD154, GITR, GM-CSF.

23. The chimeric antigen receptor according to claim 21, wherein the chimeric antigen receptor further comprises a costimulatory signaling domain.

24. The chimeric antigen receptor according to claim 23, wherein the costimulatory signaling domain comprises a costimulatory signaling domain of the following molecules: 4-1BB(CD137), CD27, CD19, CD4, CD28, ICOS(CD278), CD8α, CD8β, BAFFR, HVEM, LIGHT, KIRDS2, SLAMF7, NKp30, NKp46, CD40, CDS, ICAM-1, B7-H3, OX40, DR3, GITR, CD30, TIM1, CD2, CD7, CD226.

25. The chimeric antigen receptor according to claim 24, wherein the costimulatory signaling domain is a 4-1BB costimulatory signaling domain.

26. The chimeric antigen receptor according to claim 23, wherein the chimeric antigen receptor further comprises EF1α, T2A, tEGFR.

27. The chimeric antigen receptor according to claim 26, wherein the chimeric antigen receptor further comprises a tEGFR signal peptide.

28. The chimeric antigen receptor according to claim 27, wherein the chimeric antigen receptor is obtained by sequentially linking EF1α, a signal peptide, any one of the nanobody according to claim 1 or the humanized anti-CD7 nanobody according to claim 5, a CD8α hinge region, a CD8α transmembrane domain, a 4-1BB costimulatory signaling domain, a CD3ζ intracellular signaling domain, T2A, a tEGFR signal peptide, and tEGFR in series.

29. A chimeric antigen receptor based on a double nanobody, wherein the chimeric antigen receptor comprises any two selected from the nanobody according to claim 1 or the humanized anti-CD7 nanobody according to claim 5.

30. The chimeric antigen receptor according to claim 29, wherein the chimeric antigen receptor further comprises a transmembrane domain.

31. The chimeric antigen receptor according to claim 30, wherein the transmembrane domain comprises a transmembrane domain of the following molecules: CD8α, CD28, IgG1, IgG4, 4-1BB, PD-1, CD34, OX40, CD3ε, IL-2 receptor, IL-7 receptor, IL-11 receptor.

32. The chimeric antigen receptor according to claim 31, wherein the transmembrane domain is a CD8α transmembrane domain.

33. The chimeric antigen receptor according to claim 30, wherein the chimeric antigen receptor further comprises an intracellular signaling domain.

34. The chimeric antigen receptor according to claim 33, wherein the intracellular signaling domain comprises an intracellular signaling domain of the following molecules: CD3ζ, FcRγ, FcRβ, CD3γ, CD3δ, CD3ε, TCRζ, CD4, CD5, CD8, CD21, CD22, CD79a, CD79b, CD278, FcεRI, DAP10, DAP12, CD66d.

35. The chimeric antigen receptor according to claim 34, wherein the intracellular signaling domain is a CD3ζ intracellular signaling domain.

36. The chimeric antigen receptor according to claim 33, wherein the chimeric antigen receptor further comprises a hinge region.

37. The chimeric antigen receptor according to claim 36, wherein the hinge region comprises a hinge region of the following molecules: CD8α, CD28, IgG1, IgG4, 4-1BB, PD-1, CD34, OX40, CD3ε, IL-2 receptor, IL-7 receptor, IL-11 receptor.

38. The chimeric antigen receptor according to claim 37, wherein the hinge region is a CD8α hinge region.

39. The chimeric antigen receptor according to claim 36, wherein the chimeric antigen receptor further comprises a signal peptide.

40. The chimeric antigen receptor according to claim 39, wherein the signal peptide comprises a signal peptide of the following molecules: alpha and beta chains of T cell receptor, CD3ζ, CD3ε, CD4, CD5, CD8, CD9, CD28, CD16, CD22, CD33, CD37, CD45, CD64, CD80, CD86, CD134, CD137, CD154, GITR, GM -CSF.

41. The chimeric antigen receptor according to claim 39, wherein the chimeric antigen receptor further comprises a costimulatory signaling domain.

42. The chimeric antigen receptor according to claim 41, wherein the costimulatory signaling domain comprises a costimulatory signaling domain of the following molecules: 4-1BB(CD137), CD27, CD19, CD4, CD28, ICOS(CD278), CD8α, CD8β, BAFFR, HVEM, LIGHT, KIRDS2, SLAMF7, NKp30, NKp46, CD40, CDS, ICAM-1, B7-H3, OX40, DR3, GITR, CD30, TIM1, CD2, CD7, CD226.

43. The chimeric antigen receptor according to claim 42, wherein the costimulatory signaling domain is a 4-1BB costimulatory signaling domain.

44. The chimeric antigen receptor according to claim 41, wherein the chimeric antigen receptor further comprises EF1α, T2A, tEGFR.

45. The chimeric antigen receptor according to claim 44, wherein the chimeric antigen receptor further comprises a tEGFR signal peptide.

46. The chimeric antigen receptor according to claim 29, wherein, any two nanobodies selected from the nanobody according to claim 1 or the humanized anti-CD7 nanobody according to claim 5 are linked through a linking peptide Linker.

47. The chimeric antigen receptor according to claim 46, wherein the chimeric antigen receptor is obtained by sequentially linking EF1α, a signal peptide, any two selected from the nanobody according to claim 1 or the humanized anti-CD7 nanobody according to claim 5, a CD8α hinge region, a CD8α transmembrane domain, a 4-1BB costimulatory signaling domain, a CD3ζ intracellular signaling domain, T2A, a tEGFR signal peptide, and tEGFR in series.

48. The chimeric antigen receptor according to claim 47, wherein the chimeric antigen receptor is obtained by sequentially linking EF1α, a signal peptide, any one of the nanobody according to claim 1, Linker, any one of the nanobody according to claim 1, a CD8α hinge region, a CD8α transmembrane domain, a 4-1BB costimulatory signaling domain, a CD3ζ intracellular signaling domain, T2A, a tEGFR signal peptide, and tEGFR in series.

49. The chimeric antigen receptor according to claim 48, wherein, any one of the nanobody according to claim 1 is VHH03, VHH06, VHH10, or VHH12.

50. The chimeric antigen receptor according to claim 49, wherein the chimeric antigen receptor is obtained by sequentially linking EF1α, a signal peptide, VHH06 according to claim 1, Linker, VHH03 according to claim 1, a CD8α hinge region, a CD8α transmembrane domain, a 4-1BB costimulatory signaling domain, a CD3ζ intracellular signaling domain, T2A, a tEGFR signal peptide, and tEGFR in series.

51. The chimeric antigen receptor according to claim 49, wherein the chimeric antigen receptor is obtained by sequentially linking EF1α, a signal peptide, VHH06 according to claim 1, Linker, VHH12 according to claim 1, a CD8α hinge region, a CD8α transmembrane domain, a 4-1BB costimulatory signaling domain, a CD3ζ intracellular signaling domain, T2A, a tEGFR signal peptide, and tEGFR in series.

52. The chimeric antigen receptor according to claim 49, wherein the chimeric antigen receptor is obtained by sequentially linking EF1α, a signal peptide, VHH10 according to claim 1, Linker, VHH10 according to claim 1, a CD8α hinge region, a CD8α transmembrane domain, a 4-1BB costimulatory signaling domain, a CD3ζ intracellular signaling domain, T2A, a tEGFR signal peptide, and tEGFR in series.

53. The chimeric antigen receptor according to claim 49, wherein the chimeric antigen receptor is obtained by sequentially linking EF1α, a signal peptide, VHH10 according to claim 1, Linker, VHH12 according to claim 1, a CD8α hinge region, a CD8α transmembrane domain, a 4-1BB costimulatory signaling domain, a CD3ζ intracellular signaling domain, T2A, a tEGFR signal peptide, and tEGFR in series.

54. The chimeric antigen receptor according to claim 49, wherein the chimeric antigen receptor is obtained by sequentially linking EF1α, a signal peptide, VHH12 according to claim 1, Linker, VHH12 according to claim 1, a CD8α hinge region, a CD8α transmembrane domain, a 4-1BB costimulatory signaling domain, a CD3ζ intracellular signaling domain, T2A, a tEGFR signal peptide, and tEGFR in series.

55. A nucleic acid molecule, wherein the nucleic acid molecule comprises a nucleotide sequence encoding the nanobody according to claim 1, the humanized anti-CD7 nanobody according to claim 5, the chimeric antigen receptor according to claim 11, or the chimeric antigen receptor according to claim 29.

56. The nucleic acid molecule according to claim 55, wherein the nucleotide sequence is as shown in SEQ ID NO: 2, SEQ ID NO: 10, SEQ ID NO: 18, SEQ ID NO: 26, SEQ ID NO: 34, SEQ ID NO: 42, SEQ ID NO: 50, SEQ ID NO: 58, SEQ ID NO: 66, SEQ ID NO: 74, SEQ ID NO: 82, SEQ ID NO: 90, SEQ ID NO: 98, SEQ ID NO: 106, SEQ ID NO: 114, SEQ ID NO: 122, or SEQ ID NO: 130 or is a nucleotide sequence at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 2, SEQ ID NO: 10, SEQ ID NO: 18, SEQ ID NO: 26, SEQ ID NO: 34, SEQ ID NO: 42, SEQ ID NO: 50, SEQ ID NO: 58, SEQ ID NO: 66, SEQ ID NO: 74, SEQ ID NO: 82, SEQ ID NO: 90, SEQ ID NO: 98, SEQ ID NO: 106, SEQ ID NO: 114, SEQ ID NO: 122, or SEQ ID NO: 130.

57. A recombinant expression vector, wherein the recombinant expression vector comprises the nucleic acid molecule according to claim 55.

58. The recombinant expression vector according to claim 57, wherein the expression vector comprises a DNA vector, an RNA vector, a plasmid, a transposon vector, a CRISPR/Cas9 vector, or a viral vector.

59. The recombinant expression vector according to claim 58, wherein the viral vector comprises a lentiviral vector, an adenoviral vector, a retroviral vector.

60. An engineered host cell, wherein the engineered host cell expresses the nanobody according to claim 1, the humanized anti-CD7 nanobody according to claim 5, the chimeric antigen receptor according to claim 11, or the chimeric antigen receptor according to claim 29;
and the engineered host cell further comprises a population of engineered host cells.

61. The engineered host cell according to claim 60, wherein the engineered host cell comprises the recombinant expression vector according to claim 57.

62. The engineered host cell according to claim 61, wherein the engineered host cell comprises an engineered immune cell.

63. The engineered host cell according to claim 62, wherein the engineered immune cell comprises a T cell, a NK cell, an iNKT cell, a CTL cell, a monocyte, a macrophage, a dendritic cell, a NKT cell or any combination thereof.

64. A conjugate, wherein the conjugate comprises the nanobody according to claim 1 or the humanized anti-CD7 nanobody according to claim 5, and a modification moiety connected to the nanobody, and the modification moiety comprises a detectable label, a therapeutic agent.

65. The conjugate according to claim 64, wherein the detectable label comprises an enzyme, a radionuclide, a fluorescent dye, a luminescent substance, biotin.

66. The conjugate according to claim 64, wherein the therapeutic agent comprises a drug with an anti-tumor activity or a cytotoxic agent.

67. A pharmaceutical composition, wherein the pharmaceutical composition comprises the nanobody according to claim 1, the humanized anti-CD7 nanobody according to claim 5, the chimeric antigen receptor according to claim 11, the chimeric antigen receptor according to claim 29, the nucleic acid molecule according to claim 55, the recombinant expression vector according to claim 57, the engineered host cell according to claim 60, or the conjugate according to claim 64.

68. A kit, wherein the kit comprises the nanobody according to claim 1, the humanized anti-CD7 nanobody according to claim 5, the chimeric antigen receptor according to claim 11, the chimeric antigen receptor according to claim 29, the nucleic acid molecule according to claim 55, the recombinant expression vector according to claim 57, the engineered host cell according to claim 60, or the conjugate according to claim 64.

69. A reagent for detecting a CD7 protein or antigen fragment thereof, wherein the reagent for detecting a CD7 protein or antigen fragment thereof comprises the nanobody according to claim 1, the nanobody according to claim 5 or the conjugate according to claim 64.

70. A biological preparation, wherein the biological preparation comprises the engineered host cell according to claim 60 and/or the pharmaceutical composition according to claim 67.

71. A method of stimulating an immune response to a population of target cells or a target tissue in a mammal, wherein the method comprises the following steps: administering an effective amount of the engineered host cell according to claim 60, the conjugate according to claim 64, the pharmaceutical composition according to claim 67, or the biological preparation according to claim 70 to the mammal.

72. A method of modulating an immune response in a subject, wherein the method comprises the following steps: administering an effective amount of the engineered host cell according to claim 60, the conjugate according to claim 64, the pharmaceutical composition according to claim 67, or the biological preparation according to claim 70 to the subject.

73. A method for producing the nanobody according to claim 1 or the humanized anti-CD7 nanobody according to claim 5, wherein the method comprises the following steps: culturing the engineered host cell according to claim 60, and isolating the nanobody according to claim 1 or the humanized anti-CD7 nanobody according to claim 5 from the culture.

74. A method for specifically inhibiting CD7 activity, wherein the method comprises the following steps: introducing the nucleic acid molecule according to claim 55 into a cell of an organism, and inhibiting the CD7 activity by expressing the nanobody according to claim 1 or the humanized anti-CD7 nanobody according to claim 5.

75. A method of preventing and/or treating a CD7-associated disease or condition, wherein the method comprises the following steps: administrating an effective amount of the nanobody according to claim 1, the humanized anti-CD7 nanobody according to claim 5, the nucleic acid molecule according to claim 55, the recombinant expression vector according to claim 57, the engineered host cell according to claim 60, the conjugate according to claim 64, the pharmaceutical composition according to claim 67 or the biological preparation according to claim 70 to a subject with the CD7-associated disease or condition.

76. The method according to claim 75, wherein the CD7-associated disease or condition comprises a tumor expressing CD7.

77. The method according to claim 76, wherein the tumor is a hematological tumor of T lymphocyte lineage.

78. The method according to claim 77, wherein the tumor comprises acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

79. A method for detecting a CD7 protein or antigen fragment thereof, wherein the method comprises the following steps:
(1) obtaining a sample containing the CD7 protein or antigen fragment thereof;
(2) contacting the sample collected in step (1) with the nanobody according to claim 1, the humanized anti-CD7 nanobody according to claim 5, the conjugate according to claim 64, the kit according to claim 68 or the reagent for detecting a CD7 protein or antigen fragment thereof according to claim 69;
and (3) detecting the presence of an antibody-antigen complex.

80. The method according to claim 79, wherein the nanobody is a nanobody labeled with a label that can be used for detection.

81. The method according to claim 80, wherein the label that can be used for detection comprises a fluorescent pigment, avidin, a paramagnetic atom, a radioactive isotope.

82. The method according to claim 81, wherein the fluorescent pigment is fluorescein, rhodamine, Texas red, phycoerythrin, phycocyanin, allophycocyanin, peridinin-chlorophyll protein.

83. The method according to claim 81, wherein the avidin is biotin, avidin derived from ovalbumin, streptavidin, avidin derived from ovi vitellus, avidin analogs.

84. The method according to claim 81, wherein the radioactive isotope is radioactive iodine, radioactive cesium, radioactive iridium, radioactive cobalt.

85. A method for preparing the engineered host cell according to claim 60, wherein the method comprises the following steps: introducing the recombinant expression vector according to claim 57 into a host cell.

86. The method according to claim 85, wherein, a method for the introducing comprises lipofection, microinjection, electroporation, DNA vectors, RNA vectors, retroviral vectors, lentiviral vectors, poxviral vectors, herpes simplex viral vectors, adenoviral vectors, adeno-associated viral vectors.

87. A method for diagnosing whether a subject has a tumor expressing CD7, wherein the method comprises the following steps:
(1) providing a sample from a subject suspected of having a tumor expressing CD7;
(2) contacting the sample with the nanobody according to claim 1, the humanized anti-CD7 nanobody according to claim 5, the conjugate according to claim 64, the kit according to claim 68 or the reagent for detecting a CD7 protein or antigen fragment thereof according to claim 69;
and (3) detecting the formation of a complex comprising the nanobody and an antigen to obtain the amount of CD7 in the sample from the subject, comparing the amount of CD7 in the sample from the subject with the amount of CD7 in a known standard or reference sample, and determining whether the CD7 level in the sample from the subject falls within a tumor-associated CD7 level.

88. The method according to claim 87, wherein the sample in step (1) comprises urine, blood, serum, plasma, saliva, ascites, circulating cells, circulating tumor cells, non-tissue associated cells, tissues or histological preparations.

89. The method according to claim 87, wherein the tumor expressing CD7 is a hematological tumor of T lymphocyte lineage.

90. The method according to claim 89, wherein the tumor comprises acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

91. Use of the nanobody according to claim 1 or the humanized anti-CD7 nanobody according to claim 5 in detecting a CD7 protein or antigen fragment thereof.

92. Use of the nanobody according to claim 1 or the humanized anti-CD7 nanobody according to claim 5 in the preparation of a reagent or a kit for detecting a CD7 protein or antigen fragment thereof.

93. Use of the nucleic acid molecule according to claim 55 or the recombinant expression vector according to claim 57 in the preparation of an engineered host cell.

94. The use according to claim 93, wherein the engineered host cell is the engineered host cell according to claim 60.

95. Use of the nanobody according to any one of claims 1-4, the humanized anti-CD7 nanobody according to any one of claims 5-10, the chimeric antigen receptor according to any one of claims 11-28, the chimeric antigen receptor according to any one of claims 29-54, the nucleic acid molecule according to claim 55 or 56, the recombinant expression vector according to any one of claims 57-59, the engineered host cell according to any one of claims 60-63, the conjugate according to any one of claims 64-66, the pharmaceutical composition according to claim 67 or the biological preparation according to claim 70 in the preparation of an anti-tumor drug.

96. The use according to claim 95, wherein the anti-tumor drug comprises an anti-tumor immune cell therapy agent, an anti-tumor gene therapy drug.

97. The use according to claim 96, wherein the tumor is a tumor expressing CD7.

98. The use according to claim 97, wherein the tumor is a hematological tumor of T lymphocyte lineage.

99. The use according to claim 98, wherein the tumor comprises acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

100. Use of the nanobody according to any one of claims 1-4, the humanized anti-CD7 nanobody according to any one of claims 5-10, the chimeric antigen receptor according to any one of claims 11-28, the chimeric antigen receptor according to any one of claims 29-54, the nucleic acid molecule according to claim 55 or 56, the recombinant expression vector according to any one of claims 57-59, the engineered host cell according to any one of claims 60-63, the conjugate according to any one of claims 64-66, the pharmaceutical composition according to claim 67 or the biological preparation according to claim 70 in the preparation of a kit for preparing an immune cell for preventing and/or treating a tumor.

101. The use according to claim 100, wherein the tumor is a tumor expressing CD7.

102. The use according to claim 101, wherein the tumor is a hematological tumor of T lymphocyte lineage.

103. The use according to claim 102, wherein the tumor comprises acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

104. Use of the nanobody according to any one of claims 1-4, the humanized anti-CD7 nanobody according to any one of claims 5-10, the chimeric antigen receptor according to any one of claims 11-28, the chimeric antigen receptor according to any one of claims 29-54, the nucleic acid molecule according to claim 55 or 56, the recombinant expression vector according to any one of claims 57-59, the engineered host cell according to any one of claims 60-63, the conjugate according to any one of claims 64-66, or the pharmaceutical composition according to claim 67 in the preparation of a biological preparation for preventing and/or treating a tumor.

105. The use according to claim 104, wherein the tumor is a tumor expressing CD7.

106. The use according to claim 105, wherein the tumor is a hematological tumor of T lymphocyte lineage.

107. The use according to claim 106, wherein the tumor comprises acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

108. Use of the engineered host cell according to any one of claims 60-63 in the prevention and/or treatment of a tumor.

109. The use according to claim 108, wherein the tumor is a tumor expressing CD7.

110. The use according to claim 109, wherein the tumor is a hematological tumor of T lymphocyte lineage.

111. The use according to claim 110, wherein the tumor comprises acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

112. Use of the conjugate according to any one of claims 64-66 in the prevention and/or treatment of a tumor.

113. The use according to claim 112, wherein the tumor is a tumor expressing CD7.

114. The use according to claim 113, wherein the tumor is a hematological tumor of T lymphocyte lineage.

115. The use according to claim 114, wherein the tumor comprises acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

116. Use of the pharmaceutical composition according to claim 67 in the prevention and/or treatment of a tumor.

117. The use according to claim 116, wherein the tumor is a tumor expressing CD7.

118. The use according to claim 117, wherein the tumor is a hematological tumor of T lymphocyte lineage.

119. The use according to claim 118, wherein the tumor comprises acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).

120. Use of the kit according to claim 68 in the preparation of an immune cell for preventing and/or treating a tumor.

121. The use according to claim 120, wherein the immune cell is the engineered host cell according to claim 60.

122. Use of the reagent for detecting a CD7 protein or antigen fragment thereof according to claim 69 in the detection of a CD7 protein or antigen fragment thereof.

123. Use of the biological preparation according to claim 70 in the prevention and/or treatment of a tumor.

124. The use according to claim 123, wherein the tumor is a tumor expressing CD7.

125. The use according to claim 124, wherein the tumor is a hematological tumor of T lymphocyte lineage.

126. The use according to claim 125, wherein the tumor comprises acute myeloid leukemia (AML), acute lymphocytic leukemia (ALL), lymphoblastic lymphoma (LBL), NKT cell leukemia, peripheral T cell lymphoma (NHL), NKT cell lymphoma, anaplastic large cell lymphoma (ALCL).
